# EUROPÄISCHE PATENTSCHRIFT

(11) **EP 3 873 407 B1**
(45) Veröffentlichungstag und Bekanntmachung des Hinweises auf die Patenterteilung: **09.04.2025**
(21) Anmeldenummer: 19797680.6
(22) Anmeldetag: 31.10.2019
(51) Int. Cl.: A61K 8/31, A61K 8/34, A61K 8/41, A61K 8/58, A61Q 5/06, A61Q 5/10

(54) **ZWEIKOMPONENTENSYSTEM ZUR KÜNSTLICHEN HAARFÄRBUNG**
TWO-COMPONENT SYSTEM FOR ARTIFICIAL HAIR DYEING
SYSTÈME BICOMPOSANT POUR LA COLORATION ARTIFICIELLE DES CHEVEUX

(30) Priorität: 31.10.2018 DE 102018127182
(43) Veröffentlichungstag der Anmeldung: 08.09.2021
(73) Patentinhaber: Henkel AG & Co. KGaA, 40589 Düsseldorf (DE)
(72) Erfinder: KROHN, Rene, 22848 Norderstedt (DE); SCHULZE ZUR WIESCHE, Erik, 33619 Bielefeld (DE); POPPE, Elisabeth, 20255 Hamburg (DE)
(86) Internationale Anmeldenummer: PCT/EP2019/079779
(87) Internationale Veröffentlichungsnummer: WO 2020/089365

(56) Entgegenhaltungen:
- EP-A2- 2 168 633
- FR-A1- 2 944 965
- FR-A1- 3 060 980
- US-A1- 2010 186 177

## Beschreibung

Die vorliegende Erfindung betrifft ein Zweikomponentensystem zur künstlichen Färbung von keratinischem Material, wobei das Zweikomponentensystem getrennt voneinander ein wasserfreies Trägermedium, das ein Alkan, einen Fettalkohol, einen Alkohol, ein Amin als Alkalisierungsmittel, Farbstoffe und organische Siliciumverbindungen umfasst, sowie eine wässrige Phase umfasst, die wiederum Wasserstoffperoxid enthält. Ferner betrifft die vorliegende Erfindung die Verwendung des Zweikomponentensystem zur künstlichen Färbung oder zur künstlichen Färbung und Pflege von Haaren.

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und auch auf der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren, insbesondere solchen, die eine Pflegewirkung ausüben, um das Haar weniger zu schädigen.

Allen Färbeverfahren bedeuten eine chemische Beanspruchung der Haare. Die äußere Beanspruchung der Haare durch chemische Stoffe aus einer Vielzahl unterschiedlicher Quellen stellt die Entwicklung kosmetischer Pflegeprodukte vor Herausforderungen. Ferner sind oft wechselnde Konsumentenwünsche hinsichtlich einer bestimmten Farbe der Haare mit einer wiederkehrenden chemischen Beanspruchung der Haare verbunden. Beispielsweise beanspruchen Haarfärbungen die Haare, aufgrund dessen eine besondere, intensive Pflege nötig sein kann.

Es stellt Entwickler von Haarprodukten vor eine Herausforderung auf der einen Seite Kundenwünsche bezüglich der Farbe und Beschaffenheit zufriedenzustellen und auf der anderen Seite die Struktur, insbesondere die Oberflächenstruktur, der Haare zu schonen. Insbesondere Haarfärbungen sind mit schonenden Mitteln in der Vergangenheit schwer zu bewältigen gewesen.

Zum Schutz und zur Pflege der Haare werden im Stand der Technik siliciumorganische Verbindungen aus der Gruppe der Silane beschrieben, die mindestens eine Hydroxygruppe und/oder hydrolysierbare Gruppe umfassen. Aufgrund der Anwesenheit der Hydroxygruppen und/oder hydrolysierbaren Gruppen handelt es sich bei den Silanen um reaktive Substanzen, die in Gegenwart von Wasser hydrolysieren bzw. oligomerisieren oder polymerisieren. Die durch Anwesenheit des Wassers initiierte Oligomerisierung oder Polymerisierung der Silane führt bei Anwendung auf einem keratinischen Material letztendlich zur Ausbildung eines Films, der eine Schutzwirkung entfalten kann.

Der Fachmann kennt zum Beispiel das Dokument FR2944965-A1. Dieses Dokument betrifft ein Zweikomponentensystem zur Aufhellung oder Färbung der Haare umfassend getrennt voneinander
- eine erste Komponente, die wasserfrei ist und einen Fettkörper, ein nichtionisches Tensid und eine organische aminosubstituierte Siliciumverbindung enthält; und
- eine zweite Komponente, die ein Oxidationsmittel enthält.

Das Problem bei der Verwendung von siliciumorganischen Verbindungen ist deren Instabilität gegenüber Wasser. Wässrige Systeme zur Haarbehandlung sind somit nachteilbehaftet, wenn sie organische Silicumverbindungen als Wirksubstanzen enthalten.

Die der vorliegenden Erfindung zugrunde liegende Aufgabe besteht in der Bereitstellung eines Systems, das als Grundlage zur Herstellung eines Mittels dient, mit dem eine schonende Haarfärbung möglich ist, wobei das System eine lange Lagerhaltbarkeit zeigt. Insbesondere soll das herzustellende Mittel die Oberflächenstruktur von Haaren bei dessen Verwendung schonen oder restrukturieren.

Diese Aufgabe wird gelöst durch ein Zweikomponentensystem zur Färbung von keratinischem Material, insbesondere Haaren, wobei das Zweikomponentensystem getrennt voneinander umfasst:
- ein wasserfreies Trägermedium als erste Komponente, umfassend
   a) mindestens ein verzweigtes oder lineares C8-C30 Alkan,
   b) mindestens einen verzweigten oder linearen C10-C30 Fettalkohol,
   c) mindestens einen verzweigten oder linearen einwertigen C2-C8 Alkohol,
   d) mindestens ein verzweigtes oder lineares C2-C10 Amin als Alkalisierungsmittel,
   e) einen oder mehrere Farbstoffe zur Farbänderung des keratinischen Materials, und
   f) mindestens eine organische Siliciumverbindung, wobei die mindestens eine organische Siliciumverbindung eine Verbindung der Formel (I) und mindestens eine organische Siliciumverbindung der Formel (IV) wie nachstehend definiert enthält, und
- eine wässrige Phase als zweite Komponente, umfassend Wasser und Wasserstoffperoxid.

Der Vorteil von Zweikomponentensystemen liegt in der Bereitstellung der Möglichkeit, Stoffe, die miteinander reagieren, getrennt voneinander aufbewahren zu können. Erst kurz vor Anwendung des Zweikomponentensystems können die Komponenten miteinander kombiniert werden, um ein anwendungsbereites Mittel bereitzustellen. Im Rahmen der vorliegenden Erfindung soll somit das Merkmal "getrennt voneinander umfassend" so verstanden werden, dass die zwei Komponenten, also das wasserfreie Trägermedium und die wässrige Phase, in zwei Kompartimenten oder zwei unterschiedlichen Behältern räumlich getrennt voneinander vorliegen, mit dem Zweck, dass sie sich nicht ungewollt durchmischen zu können.

Das wasserfreie Trägermedium dient als Grundlage für die Bereitstellung einer siliciumorganischen Verbindung. In dem Trägermedium bleibt die organische Siliciumverbindung laberstabil. Das Trägermedium kann mit einer oder mehreren wässrigen Phasen als zweite Komponente kombiniert werden, die für die Haarfärbung dienende Wirkstoffe enthält und auf Wasser basiert. Dadurch dass das Trägermedium dann mit der einen oder den weiteren wässrigen Phasen erst kurz vor der Anwendung zusammengegeben wird, bleibt die Wirksubstanz siliciumorganische Verbindung bis zur Anwendung stabil.

Unter "wasserfrei" soll im Rahmen der vorliegenden Erfindung bevorzugt verstanden werden, dass dem wässrigen Trägermedium nicht Wasser zugegeben wird oder das wässrige Trägermedium nicht wasserbasiert ist. Bevorzugter liegt der Wassergehalt des wasserfreien Trägermediums bei weniger als 5 Gew.-%, noch bevorzugter bei weniger als 2 Gew.-%, am meisten bevorzugt bei weniger als 1 Gew.-%, bezogen auf das Gesamtgewicht des wasserfreien Trägermediums. Beim Vorliegen von geringen Feuchtigkeitsmengen kann ein geringer Teil der organischen Siliciumverbindung hydrolysieren und das Hydrolysat liegt im Gleichgewicht mit freiem Wasser vor. Diese Menge an Wasser liegt bevorzugt in den oben genannten Mengen vor.

Unter einem keratinischen Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter einem keratinischen Material das menschliche Haar, die menschliche Haut und menschliche Nägel, insbesondere Finger- und Fußnägel, verstanden. Ganz besonders bevorzugt wird unter keratinischem Material das menschliche Haar, insbesondere Kopf- und/oder Barthaare, verstanden.

Das wasserfreie Trägermedium des Zweikomponentensystems enthält mindestens eine organische Siliciumverbindung, nämlich mindestens eine organische Siliciumverbindung der Formel (I) und mindestens eine organische Siliciumverbindung der Formel (IV) wie nachstehend definiert, die stabilisiert werden sollen. Durch den Einsatz von organischen Siliciumverbindungen in dem wasserfreien Träger sind die organischen Siliciumverbindungen vor Hydrolyse geschützt.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist.

Die Wirkung der organischen Siliciumverbindungen betrifft den Schutz und die Pflege der Haare, insbesondere der Haaroberfläche, bei der Verwendung des Zweikomponentensystems zur Färbung von Haaren. Die hydrolysierten Siliciumverbindungen, wenn mit der wässrigen Phase zusammengebracht, bilden einen Schutzfilm auf der Haaroberfläche und haben somit einen "Repair"-Effekt.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die WasserstoffAtome ganz oder teilweise durch organische Gruppen wie beispielsweise (substituierte) Alkylgruppen und/oder Alkoxygruppen ersetzt. In den organischen Silanen kann auch ein Teil der Wasserstoffatome durch Hydroxygruppen ersetzt sein.

Das wasserfreie Trägermedium enthält mindestens eine organische Siliciumverbindung der Formel (I) und mindestens eine organische Siliciumverbindung der Formel (IV). In einer bevorzugten Ausführungsform enthält das wasserfreie Trägermedium zusätzlich zu den organischen Siliciumverbindungen der Formel (I) und (IV) eine weitere organische Siliciumverbindung der Formel (II).

Die Verbindungen der Formeln (I) und (II) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

Die Siliciumverbindungen der Formel (I) und (II) sind:

R₁R₂N-L-Si(OR₃)ₐ(R₄)_{b} (I),

wobei
- R₁, R₂ beide für ein Wasserstoffatom stehen,
- L für eine lineare, zweibindige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und
- b für die Zahl 0 steht,

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- R5, R5', R5" unabhängig voneinander für ein Wasserstoffatom oder für eine C₁-C₆-Alkylgruppe stehen,
- R6, R6' und R6" unabhängig voneinander für eine C₁-C₆-Alkylgruppe stehen,
- A, A', A", A‴ und Aʺʺ unabhängig voneinander für eine lineare oder verzweigte, zweibindige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (III) stehen

- (Aʺʺ)-Si(Rₑ")_{d}"(OR₅")_{c}" (III),

- c, für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
- mit der Maßgabe, dass mindestens einer der Reste aus e, f, g und h von 0 verschieden ist.

Die Substituenten R₁, R₂, R₃, R₄, R₅, R₅', R₅", R₆, R₆', R₆", R₇, R₈, L, A, A', A", A‴ und Aʺʺ in den Verbindungen der Formel (I) und (II) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-C₁-C₆-alkyl-gruppe sind die Aminomethylgruppe, die 2-Aminoethylgruppe, die 3-Aminopropylgruppe. Die 2-Aminoethylgruppe ist besonders bevorzugt. Beispiele für eine lineare zweibindige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweibindige Alkylengruppen auch verzweigt sein. Beispiele für verzweigte, zweibindige C₃-C₂₀-Alkylengruppen sind (-CH₂-CH(CH₃)-) und (-CH₂-CH(CH₃)-CH₂-).

Die organische Siliciumverbindung der Formel (IV) ist:

R₉Si(OR₁₀)ₖ(R₁₁)ₘ (IV)

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3-k steht.

Die beste Pflege von beanspruchtem Haar konnte erhalten werden, wenn das wasserfreie Trägermedium mindestens eine organische Siliciumverbindung der Formel (I) oder Formel (II) enthält, in denen die Reste R₃, R₄ unabhängig voneinander für eine Methylgruppe oder für eine Ethylgruppe stehen.

Besonders gut geeignete organische Siliciumverbindungen der Formel (I) sind
- (3-Aminopropyl)triethoxysilan 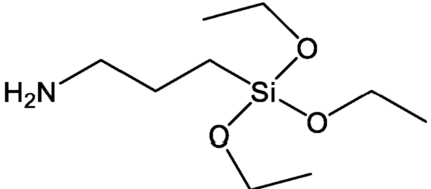
- (3-Aminopropyl)trimethoxysilan 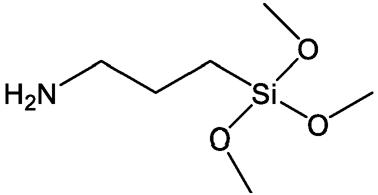
- (2-Aminoethyl)triethoxysilan 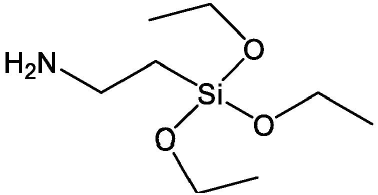
- (2-Aminoethyl)trimethoxysilan

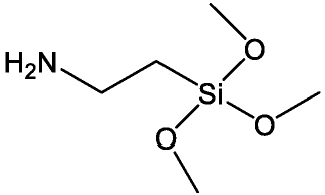

Die vorgenannten organische Siliciumverbindung der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erhältlich.

Im Rahmen einer weiteren Ausführungsform enthält das wasserfreie Trägermedium mindestens eine organische Siliciumverbindung der Formel (II)

(R₅O)_{c}(Rₑ)_{d}Si-(A)ₑ-[NR₇-(A')]_{f}-[O-(A")]_{g}-[NRₑ-(A‴)]ₕ-Si(R₆')_{d'}(OR₅')_{c'} (II).

Die siliciumorganischen Verbindungen der Formel (II) tragen jeweils an ihren beiden Enden die Siliciumhaltigen Gruppierungen (R₅O)_{c}(R₆)_{d}Si- und -Si(R₆')_{d'}(OR₅')_{c'}.

Im Mittelteil des Moleküls der Formel (II) befinden sich die Gruppierungen -(A)ₑ- und -[NR₇-(A')]_{f}- und -[O-(A")]_{g}- und -[NRₐ-(A‴)]ₕ-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine organischen Siliciumverbindung der Formel (II) mindestens eine Gruppierung aus der Gruppe aus -(A)- und -[NR₇-(A')]- und -[O-(A")]- und -[NR₈-(A‴)]-.

In den beiden endständigen Struktureinheiten (R₅O)_{c}(R₆)_{d}Si- und - Si(R₆')_{d'}(OR₅')_{c}, stehen die Reste R5, R5', R5" unabhängig voneinander für ein Wasserstoffatom oder für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c' für die Zahl 2 steht, dann ist d' gleich 1. Wenn c' für die Zahl 1 steht, dann ist d' gleich 2.

Sehr bevorzugte Trägermedien enthalten eine organische Siliciumverbindung, bei der die Reste c und c' beide für die Zahl 3 stehen. In diesem Fall stehen d und d' beide für die Zahl 0.

In einer weiteren bevorzugten enthält das wasserfreie Trägermedium des Zweikomponentensystems mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die organischen Siliciumverbindung der Formel (IIa)

(R₅O)₃Si-(A)ₑ-[NR₇-(A')]_{f}-[O-(A")_{g}-[NR₈-(A‴)]ₕ-Si(OR₅')₃ (IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NRₑ-(A‴)]ₕ- sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erhöhung der Pflegewirkung erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die organischen Siliciumverbindung der Formel (IIb)

(R₅O)_{c}(R₆)_{d}Si-(A)-[NR₇-(A')]-Si(R₆')_{d'}(OR₅')_{c'} (IIb).

Die Reste A, A', A", A‴ und A"" stehen unabhängig voneinander für eine lineare oder verzweigte, zweibindige C₁-C₂₀-Alkylengruppe. Bevorzugt stehen die Reste A, A', A", A‴ und Aʺʺ unabhängig voneinander für eine lineare, zweibindige C₁-C₂₀-Alkylengruppe. Weiter bevorzugt stehen die Reste A, A', A", A‴ und Aʺʺ unabhängig voneinander für eine lineare zweibindige C₁-C₆-Alkylengruppe. Besonders bevorzugt stehen die Reste A, A', A", A‴ und A"" unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und Aʺʺ für eine Propylengruppe (-CH₂-CH₂-CH₂-).

Wenn der Rest f für die Zahl 1 steht, dann enthält die organische Siliciumverbindung der Formel (II) eine strukturelle Gruppierung -[NR₇-(A')]-.

Wenn der Rest h für die Zahl 1 steht, dann enthält die organische Siliciumverbindung der Formel (II) eine strukturelle Gruppierung -[NR₈-(A‴)]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III)

- (Aʺʺ)-Si(Rₑ")_{d}"(OR₅")_{c}" (III).

Ganz besonders bevorzugt stehen die Reste R₇ und R₈ unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (III).

Wenn der Rest f für die Zahl 1 steht und der Rest h für die Zahl 0 steht, enthält die organische Siliciumverbindung die Gruppierung [NR₇-(A')], aber nicht die Gruppierung -[NR₈-(A‴)]. Steht nun der Rest R7 für eine Gruppierung der Formel (III), so enthält das wasserfreie Trägermedium eine organische Siliciumverbindung mit 3 reaktiven Silan-Gruppen.

In einer weiteren bevorzugten enthält das wasserfreie Trägermedium des Zweikomponentensystems mindestens eine organische Siliciumverbindung der Formel (II)

(R₅O)_{c}(R₆)_{d}Si-(A)ₑ-[NR₇₋(A')]_{f}-[O-(A")]_{g}-[NR₈-(A‴)]ₕ-Si(R₆')_{d'}(OR₅')_{c'} (II),

wobei
- e und f beide für die Zahl 1 stehen,
- g und h beide für die Zahl 0 stehen,
- A und A' unabhängig voneinander für eine lineare, zweibindige C₁-C₆-Alkylengruppe stehen
   und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

In einer weiteren bevorzugten Ausführungsform enthält das wasserfreie Trägermedium des Zweikomponentensystems mindestens eine organische Siliciumverbindung der Formel (II), wobei
- e und f beide für die Zahl 1 stehen,
- g und h beide für die Zahl 0 stehen,
- A und A' unabhängig voneinander für eine Methylengruppe (-CH₂-), eine Ethylengruppe (-CH₂-CH₂-) oder eine Propylengruppe (-CH₂-CH₂-CH₂) stehen,
   und
- R₇ für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

Zur Lösung der Aufgabenstellung gut geeignete organische Siliciumverbindungen der Formel (II) sind
- 3-(Trimethoxysilyl)-N-[3-(trimethoxysilyl)propyl]-1-propanamin 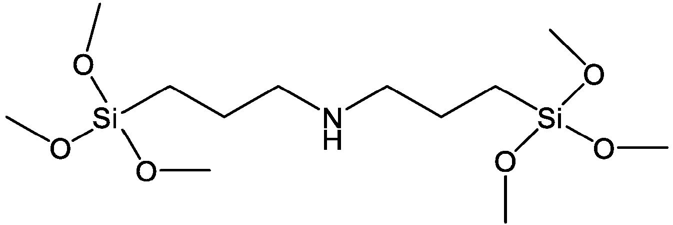
- 3-(Triethoxysilyl)-N-[3-(triethoxysilyl)propyl]-1-propanamin 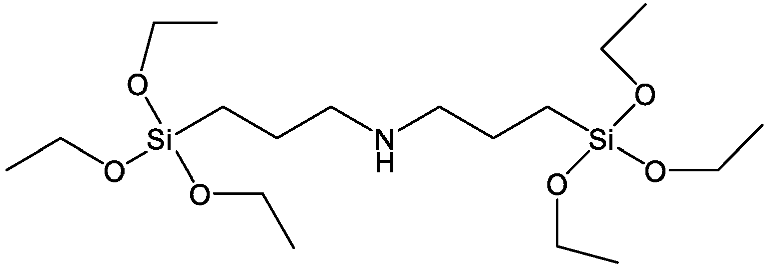
- N-Methyl-3-(trimethoxysilyl)-N-[3-(trimethoxysilyl)propyl]-1-propanamin 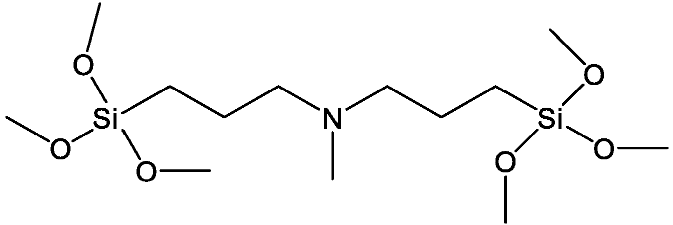
- N-Methyl-3-(triethoxysilyl)-N-[3-(triethoxysilyl)propyl]-1-propanamin 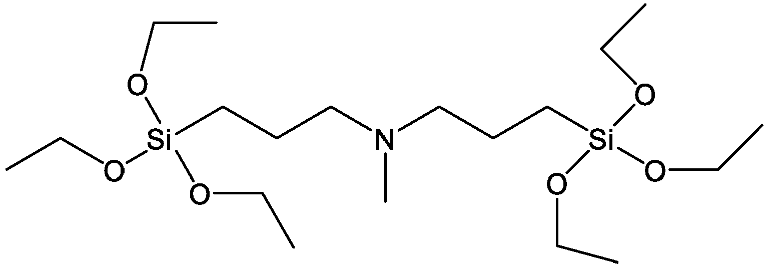
- 2-[Bis[3-(trimethoxysilyl)propyl]amino]-ethanol 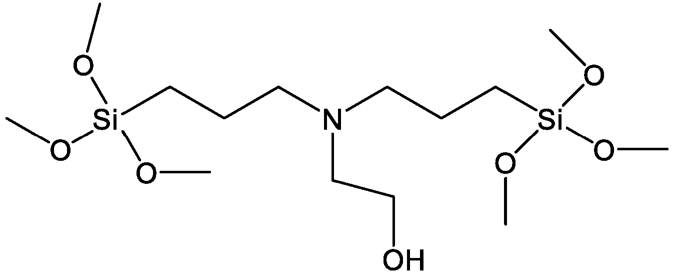
- 2-[Bis[3-(triethoxysilyl)propyl]amino]-ethanol 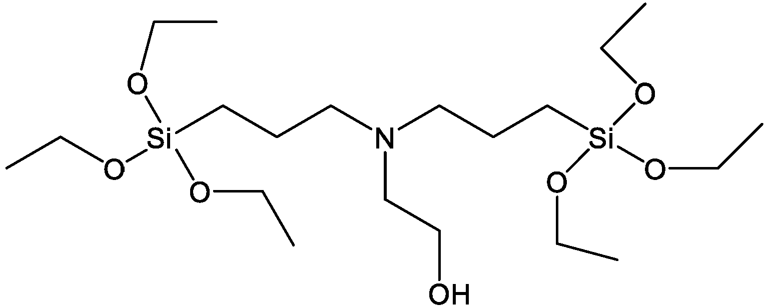
- 3-(Trimethoxysilyl)-N,N-bis[3-(trimethoxysilyl)propyl]-1-propanamin 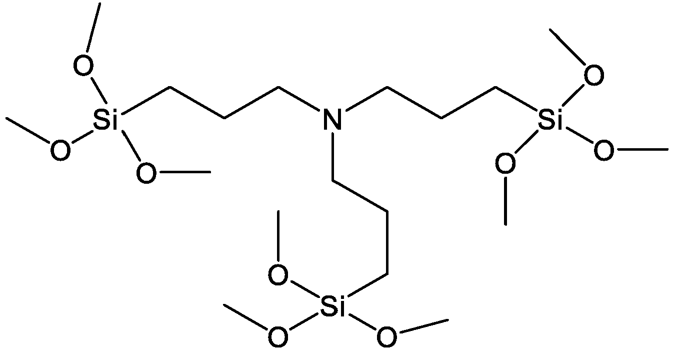
- 3-(Triethoxysilyl)-N,N-bis[3-(triethoxysilyl)propyl]-1-propanamin 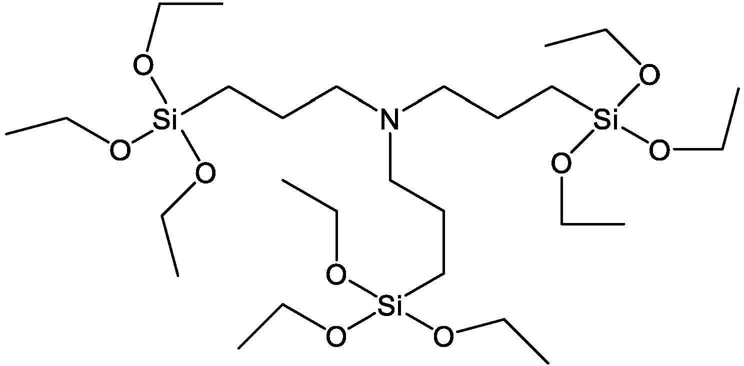
- N1,N1-Bis[3-(trimethoxysilyl)propyl]-1,2-ethanediamin, 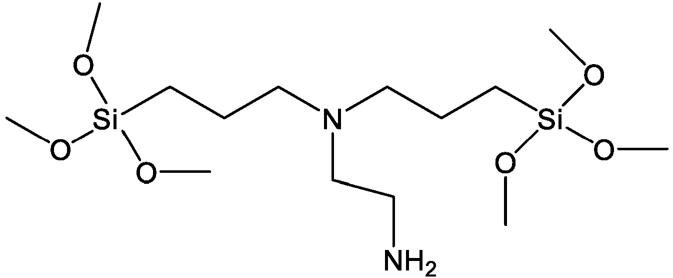
- N1,N1-Bis[3-(triethoxysilyl)propyl]-1,2-ethanediamin, 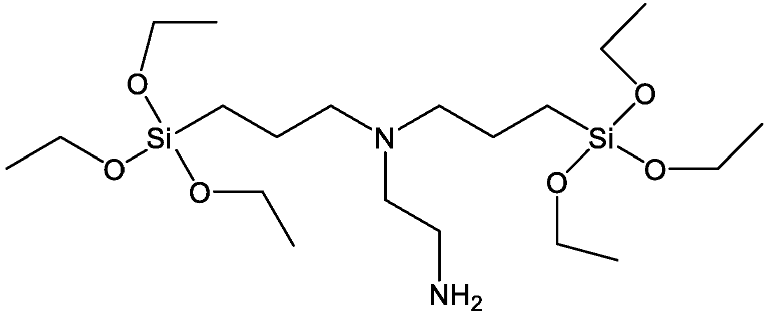
- N,N-Bis[3-(trimethoxysilyl)propyl]-2-propen-1-amin 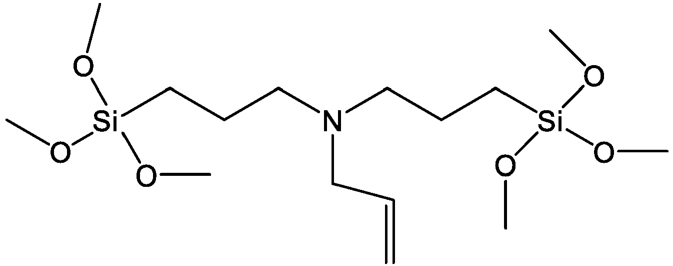
- N,N-Bis[3-(triethoxysilyl)propyl]-2-propen-1-amin

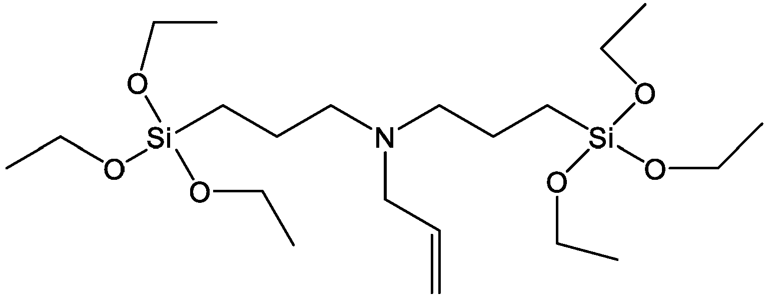

Die vorgenannten organische Siliciumverbindung der Formel (II) sind kommerziell erhältlich.

Bis(trimethoxysilylpropyl)amin mit der CAS-Nummer 82985-35-1 kann beispielsweise von Sigma-Aldrich käuflich erworben werden.

Bis[3-(triethoxysilyl)propyl]amin, auch bezeichnet als 3-(Triethoxysilyl)-N-[3-(triethoxysilyl)propyl]-1-propanamin, mit der CAS-Nummer 13497-18-2 kann zum Beispiel von Sigma-Aldrich käuflich erworben werden oder ist im Handel unter der Produktbezeichnung Dynasylan 1122 von Evonik erhältlich.

N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

In eine bevorzugten Ausführungsform enthält das wasserfreie Trägermedium des Zweikomponentensystems zusätzlich zu den organischen Siliciumverbindungen der Formel (I) und (IV) mindestens eine weitere organische Siliciumverbindung der Formel (II).

In den organischen Siliciumverbindungen der Formel (IV) steht der Rest R₉ für eine C₁-C₁₂-Alkylgruppe.

Diese C₁-C₁₂-Alkylgruppe ist gesättigt und kann linear oder verzweigt sein. Bevorzugt steht R9 für eine lineare C₁-C₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe oder eine n-Dodecylgruppe. Besonders bevorzugt steht R₉ für eine Methylgruppe, eine Ethylgruppe oder eine n-Octylgruppe.

In den organischen Siliciumverbindungen der Formel (IV) steht der Rest R₁₀ für ein Wasserstoffatom oder eine C₁-C₆-Alkylgruppe. Besonders bevorzugt steht R₁₀ für eine Methylgruppe oder für eine Ethylgruppe.

In den organischen Siliciumverbindungen der Formel (IV) steht der Rest R₁₁ für eine C₁-C₆-Alkylgruppe.

Besonders bevorzugt steht R₁₁ für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Es hat sich als sehr vorteilhaft erwiesen, dass das wasserfreie Trägermedium mindestens eine organische Siliciumverbindung der Formel (IV) enthält, in welcher der Rest k für die Zahl 3 steht. In diesem Fall steht der Rest m für die Zahl 0.

Zur Lösung der Aufgabenstellung besonders gut geeignete organische Siliciumverbindungen der Formel (IV) sind
- Methyltrimethoxysilan 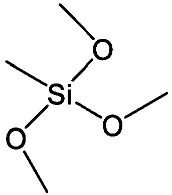
- Methyltriethoxysilan 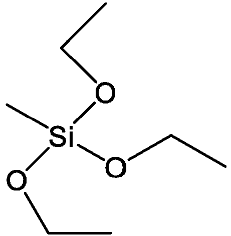
- Ethyltrimethoxysilan 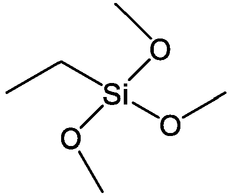
- Ethyltriethoxysilan 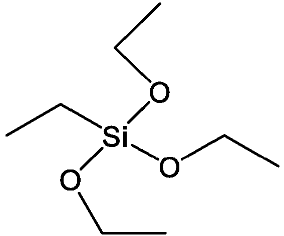
- n-Hexyltrimethoxysilan 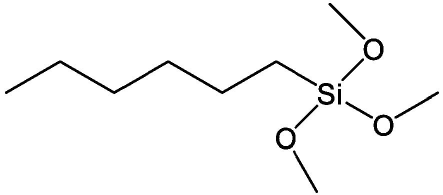
- n-Hexyltriethoxysilan 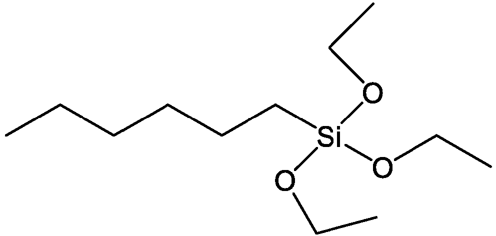
- n-Octyltrimethoxysilan 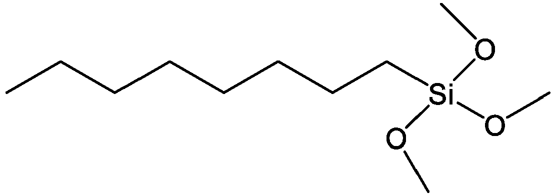
- n-Octyltriethoxysilan 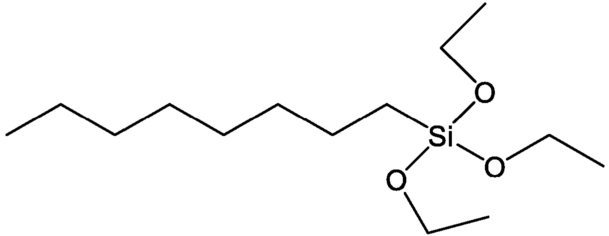
- n-Dodecyltrimethoxysilan und/oder 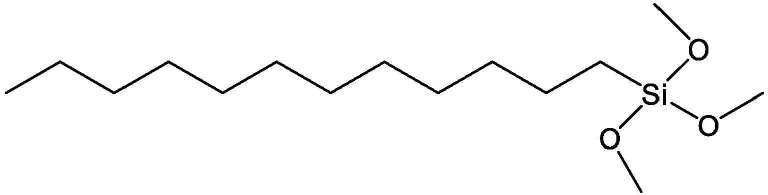
- n-Dodecyltriethoxysilan 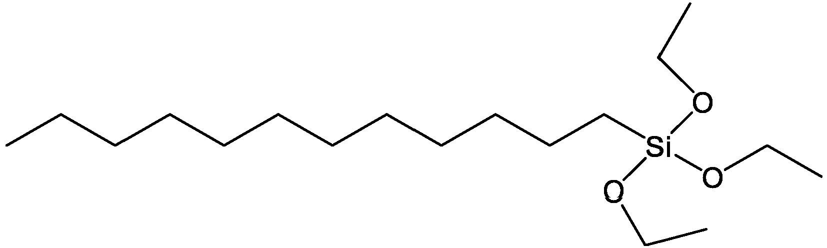
sowie Propyltrimethoxysilan und/oder Propyltriethoxysilan.

Bei den zuvor beschriebenen organischen Siliciumverbindungen handelt es sich um reaktive Verbindungen.

Es hat sich herausgestellt, dass auf dem keratinischen Material besonders stabile und gleichmäßige Filme erhalten werden konnten, wenn das wasserfreie Trägermedium zwei strukturell voneinander verschiedene organische Siliciumverbindungen enthält.

Das wasserfreie Trägermedium ist daher dadurch gekennzeichnet, dass es mindestens eine organische Silicumverbindungen der Formel (I) und mindestens eine organische Silicumverbindung der Formel (IV) enthält.

In einer explizit ganz besonders bevorzugten Ausführungsform ist ein wasserfreies Trägermedium dadurch gekennzeichnet, dass es mindestens eine organische Silicumverbindungen der Formel (I) enthält, welche aus der Gruppe aus (3-Aminopropyl)triethoxysilan und (3-Aminopropyl)trimethoxysilan ausgewählt ist, und zusätzlich mindestens eine organische Silicumverbindungen der Formel (IV) enthält, welche aus der Gruppe bestehend aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan und Hexyltriethoxysilan ausgewählt ist.

In einer weiteren bevorzugten Ausführungsform ist ein wasserfreies Trägermedium dadurch gekennzeichnet, dass das wasserfreie Trägermedium - bezogen auf das Gesamtgewicht des wasserfreie Trägermediums - enthält:
- 0,5 bis 5 Gew.-% mindestens einer ersten organischen Siliciumverbindung, die ausgewählt ist aus der Gruppe aus (3-Aminopropyl)trimethoxysilan, (3-Aminopropyl)triethoxysilan, (2-Aminoethyl)trimethoxysilan und (2-Aminoethyl)triethoxysilan, und
- 3,2 bis 10 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan und Dodecyltriethoxysilan.

Bereits der Zusatz geringer Wassermengen führt bei organischen Siliciumverbindungen mit mindestens einer hydrolysierbaren Gruppe zur Hydrolyse. Die Hydrolyseprodukte und/oder organischen Siliciumverbindungen mit mindestens einer Hydroxygruppe können in einer Kondensationsreaktion miteinander reagieren. Aus diesem Grund können sowohl die siliciumorganischen Verbindungen mit mindestens einer hydrolysierbaren Gruppe als auch deren Hydrolyse- und/oder Kondensationsprodukte in dem wasserfreie Trägermedium enthalten sein. Bei Verwendung von siliciumorganischen Verbindungen mit mindestens einer Hydroxylgruppe können sowohl die organischen Siliciumverbindungen mit mindestens einer Hydroxylgruppe als auch deren Kondensationsprodukte in dem wasserfreiem Trägermedium enthalten sein.

Unter einem Kondensationsprodukt wird ein Produkt verstanden, dass durch Reaktion von mindestens zwei organischen Siliciumverbindungen mit jeweils mindestens einer Hydroxylgruppen oder hydrolysierbaren Gruppen pro Molekül unter Abspaltung von Wasser und/oder unter Abspaltung von einem Alkanol entsteht. Die Kondensationsprodukte können beispielsweise Dimere, aber auch Trimere oder Oligomere sein, wobei die Kondensationsprodukte mit den Monomeren im Gleichgewicht stehen. Abhängig von der eingesetzten oder in der Hydrolyse verbrauchten Wassermenge verschiebt sich das Gleichgewicht von monomerer organischen Siliciumverbindungen zu Kondensationsprodukt.

Im Rahmen der vorliegenden Erfindung sind Angaben in Gew.-% - falls nicht anders angegeben - immer bezogen auf das Gesamtgewicht des wasserfreien Trägermediums. Gegebenenfalls sind die Angaben in Gew.-% bezogen auf das Gesamtgewicht der wässrigen Phase.

Die zweite Komponente des Zweikomponentensystems der vorliegenden Erfindung ist eine wässrige Phase. Zwingend enthält diese zweite Komponente Wasser und Wasserstoffperoxid.

In der vorliegenden Erfindung führt das Wasserstoffperoxid in der wässrigen Phase des Zweikomponentensystems nach Vermengen von wässriger Phase und wasserfreiem Trägermedium dazu, dass das Zweikomponentensystem zur Haarverformung eingesetzt werden kann.

Als Komponenten des wasserfreien Trägermedium sind zwingend fünf Komponenten in dem wasserfreien Trägermedium enthalten: mindestens ein verzweigtes oder lineares C8-C30 Alkan, mindestens einen verzweigten oder linearen C10-C30 Fettalkohol, mindestens einen verzweigten oder linearen einwertigen C2-C8 Alkohol, mindestens ein verzweigtes oder lineares C2-C10 Amin als Alkalisierungsmittel, und einen oder mehrere Farbstoffe zur Farbänderung des keratinischen Materials. Im Zuge der zu dieser Erfindung führenden Arbeiten hat sich herausgestellt, dass es zur Erzielung einer besonders guten Pflegewirkung insbesondere von Vorteil ist, wenn die fünf Komponenten zusammen mit den organischen Siliciumverbindungen, beispielsweise 3-(Triethoxysilyl)-N-[3-(triethoxysilyl)propyl]-1-propanamin, d.h. einem Bis(triethoxysilylpropyl)amin, in dem wasserfreien Trägermedium kombiniert wird mit der wässrigen Phase. Durch den Ausschluss von Wasser in dem wasserfreien Trägermedium wird die organische Siliciumverbindung vor verfrühter Hydrolyse geschützt und erst bei Bedarf durch Mischen mit einer Wasserphase zu einer Haarpflegeemulsion umgewandelt, welche die organische Siliciumverbindung erst kurz vor der Applikation mittels Hydrolysereaktion aktiviert. Es wurde überraschenderweise gefunden, dass die Kombination aus Bis(triethoxysilylpropyl)amin und den fünf zwingend in dem wasserfreien Trägermedium enthaltenen Komponenten die kosmetische Akzeptanz steigert. Das Haar ist weich, die Kämmbarkeit deutlich gesteigert und die Haaroberfläche ist bei dem chemisch behandelten Haar hydrophober. Gleichzeitig wird eine schonende Färbung der Haare erzielt.

Der Begriff "Farbstoff zur Farbveränderung" wird im Rahmen dieser Erfindung für eine durch Einsatz von Pigmenten und/oder direktziehenden Farbstoffen hervorgerufene Farbgebung des Keratinmaterials, insbesondere des Haares, verwendet. Bei dieser Färbung lagern sich die vorgenannten farbgebenden Verbindungen in einem besonders homogenen und glatten Film an der Oberfläche des Keratinmaterials ab oder diffundieren in die Keratinfaser hinein. Es kann unter dem Begriff "Farbstoff zur Farbveränderung" auch ein Pigment verstanden werden. Gemäß bevorzugter Ausführungsformen der vorliegenden Erfindung umfasst das erfindungsgemäße Zweikomponentensystem auch ein Pigment.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L. Geeignete Farbpigmente können anorganischen und/oder organischen Ursprungs sein.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

Gemß bevorzugter Ausführungsformen der vorliegenden Erfindung ist das Alkan in dem Zweikomponentensystem ein C10-C24 Alkan, bevorzugt ein C12-C18 Alkan und bevorzugter ein C14-C16 Alkan ist. Bevorzugt ist das Alkan in einer Menge von 1 bis 50 Gew.-%, bevorzugt 2 bis 45 Gew.-%, bevorzugter 3 bis 40 Gew.-%, noch bevorzugter 4 bis 35 Gew.-%, bezogen auf das Gesamtgewicht des wasserfreien Trägermediums, in dem wasserfreien Trägermedium enthalten ist. Mit einer Bezeichnung Cx bis Cy ist stets gemeint, dass die Kohlenwasserstoffkette x bis y Kohlenstoffatome aufweist.

In einer ganz besonders bevorzugten Ausführungsform der vorliegenden Erfindung ist der wasserfreie Träger ein wasserfreies Konzentrat. In der Ausführungsform liegt die Menge an Alkan in dem wasserfreien Träger bei 5 bis 90 Gew.-%, bevorzugt 10 bis 70 Gew.-%, bevorzugter 15 bis 50 Gew.-%.

Gemäß einer weiteren bevorzugten Ausführungsform der vorliegenden Erfindung ist der Fettalkohol ein C12-C24 Fettalkohol, bevorzugt ein C14-C18 Fettalkohol, wobei der Fettalkohol bevorzugt in einer Menge von 5 bis 50 Gew.-%, bevorzugter 6 bis 45 Gew.-%, noch bevorzugter 7 bis 40 Gew.-%, am meisten bevorzugt 8 bis 35 Gew.-%, bezogen auf das Gesamtgewicht des wasserfreien Trägermediums, in dem wasserfreien Trägermedium enthalten ist.

Gemäß einer weiteren bevorzugten Ausführungsform der vorliegenden Erfindung ist der Alkohol ein C3-C6 Alkohol, bevorzugt ein C4-C5 Alkohol, wobei der Alkohol bevorzugt in einer Menge von 4 bis 50 Gew.-%, bevorzugt 6 bis 45 Gew.-%, bevorzugter 8 bis 40 Gew.-%, noch bevorzugter 10 bis 30 Gew.-%, bezogen auf das Gesamtgewicht des wasserfreien Trägermediums, in dem wasserfreien Trägermedium enthalten ist. Unter einem einwertigen Alkohol ist ein Alkohol mit nur einer OH-Funktion zu verstehen.

Gemäß einer weiteren bevorzugten Ausführungsform der vorliegenden Erfindung ist das verzweigte oder lineare Amin ein C3-C8, bevorzugter ein C4-C6 Amin. Das Amin ist bevorzugt in einer Menge von 0,5 bis 10 Gew.-%, bevorzugter von 0,75 bis 7,5 Gew.-%, noch bevorzugter von 1 bis 5 Gew.-%, bezogen auf das Gesamtgewicht des wasserfreien Trägers, im wasserfreien Träger enthalten.

Gemäß einer weiteren bevorzugten Ausführungsform der vorliegenden Erfindung ist ein Farbstoff oder sind mehrere Farbstoffe ausgewählt aus der folgenden Gruppe von Farbstoffen in dem wasserfreien Träger enthalten:
1,7-NAPHTHALENEDIOL, m-PHENYLENEDIAMINE, TOLUENE-2,5-DIAMINE SULFATE, 2,4-DIAMINOANISOL, p-PHENYLENEDIAMINE HCl, p-PHENYLENEDIAMINE (free base), 2-CHLORO-p-PHENYLENEDIAMINE, N-PHENYL-p-PHENYLENEDIAMINE, RESORCINOL, 4-CHLORORESORCINOL, o-AMINOPHENOL, m-AMINOPHENOL, p-AMINOPHENOL, 1-NAPHTHOL, 1,5-NAPHTHALENEDIOL, 2,7-NAPHTHALENEDIOL, HYDROQUINONE, p-METHYLAMINOPHENOL, p-METHYLAMINOPHENOLSULFATE, HYDROXYBENZOMORPHOLINE, 4-AMINO-2-HYDROXYTOLUENE, 2-METHYL-5-HYDROXYETHYLAMINOPHENOL, 1,2,4-TRIHYDROXYBENZENE, PHENYL METHYL PYRAZOLONE, 2,4-DIAMINOPHENOXYETHANOL HCl oder SO4, 3-AMINO-2,4-DICHLOROPHENOL HCl, 2-METHYLRESORCINOL, N,N-BIS(2-HYDROXYETHYL)-p-PHENYLENEDIAMINE SULFATE, TETRAAMINOPYRIMIDINE SULFATE, 4-AMINO-m-CRESOL, 6-AMINO-m-CRESOL, 1,3-BIS-(2,4-DIAMINOPHENOXY)PROPANE x 4 HCl, HYDROXYETHYL-p-PHENYLENEDIAMINE SULFATE, 2-AMINO-4-HYDROXYETHYLAMINOANISOLE SULFATE, 4,4-DIAMINODIPHENYLAMINE SULFATE, 5-AMINO-6-CHLORO-o-CRESOL, HYDROXYETHYL-3,4-METHYLENEDIOXYANILINE HCl, 2,6-DIHYDROXY-3,4-DIMETHYLPYRIDINE, 2,6-DIMETHOXY-3,5-PYRIDINEDIAMIE x (2) HCl, DIHYDROXYINDOLE, 2-AMINOMETHYL-p-AMINOPHENOL HCl, 2,4-DIAMINO-5-METHYLPHENETOL HCl, 5-AMINO-4-CHLORO-o-CRESOL HCl, HYDROXYPROPYL BIS( N-HYDROXYETHYL-p-PHENYLENEDIMAMINE) HCl, 6-HYDROXYINDOLE, ISATIN, 6-METOXY-2-METHYLAMINO-3-AMINOPYRIDINE HCl (HC BLUE 7), 2-AMINO-3-HYDROXYPYRIDINE, 2-DIMETHYLAMINO-5-AMINOPYRIDINE x2HCL, 6-METHOXY-2,3-PYRIDINEDIAMINE HCL, 2,6-DIAMINO PYRIDINE, 2,6-DIHYDROXYETHYLAMINOTOLUENE, 2,5,6-TRIAMINO-4_PYRIMIDINOL SULFATE, DIHYDROXYINDOLINE HBr, 1-ACETOXY-2-METHYLNAPHTHALENE, 1-HYDROXYETHYL 4,5-DIAMINOPYRAZOLE SULFATE ,2,2'-METHYLENEBIS 4-AMINOPHENOL HCl, 2-METHYL-1-NAPHTHOL, 4-Formyl 1-methylchinolinium-p-Toluene sulfonate, 2-AMINO-5-ETHYLPHENYL HCL, 2,3-DIAMINODIHYDROXY PYRAZOLOPYRAZOLONE DIMETHOSULFONATE, 2-METHOXY-METHYL-p-PHENYLENEDIAMINE, HYDROXYETHOXY AMINOPYRAZOLOPYRIDINE HCL, 3-AMINO-2,6-DIMETHYLPHENOL, 1-HEXYL 4,5-DIAMINO PYRAZOLE, SULFATE, DIMETHYLPIPERAZINIUM AMINOPYRAZOLO PYRIDINE, METHYLIMIDAZOLIUM p-PHENYLENEDIAMINE, ACID YELLOW 1, DISPERSE RED 17,BASIC BROWN 17, ACID BLACK 52, ACID BLACK 1, DISPERSE VIOLET 4, 4-NITRO-o-PHENYLENDIAMINE, 2-NITRO-P-PHENYLENEDIAMINE, 2-AMINO-4-NITROPHENOL, 2-AMINO-5-NITROPHENOL, PICRAMIC ACID and SODIUM PICRAMATE, HC RED NO. 13, N,N'-BIS(2-HYDROXYETHYL)-2-NITRO-p-PHENYLENEDIAMINE, HC YELLOW No 5, HC RED NO. 7, HC BLUE NO. 2, HC YELLOW NO. 4, HC YELLOW NO. 2, HC ORANGE NO. 1, HC RED NO. 1, HC RED NO. 3, 4-AMINO-3-NITROPHENOL, 2-HYDROXYETHYLAMINO-5-NITROANISOLE, 3-NITRO-p-HYDROXYETHYLAMINOPHENOL, 3-METHYLAMINO-4-NITROPHENOXYETHANOL, 2-NITRO-5-GLYCERYL METHYLANILINE, HC VIOLET no 1, HC ORANGE no 2, HC YELLOW No 9, 4-NITROPHENYLAMINOETHYLUREA, HC RED NO. 10 + HC RED NO. 11, 2-HYDROXYETHYL PICRAMIC ACID, HC BLUE NO. 12 als HCl, HC YELLOW NO. 6, HYDROXYETHYL-2-NITRO-p-TOLUIDINE, HC YELLOW NO. 12, HC BLUE No 11, HC YELLOW no 7, HC YELLOW no 10, 4-AMINO-2-NITRO-DIPHENYL-AMINE-2'-CARBOXYLIC ACID, 2-CHLORO-6-ETHYLAMINO-4-NITROPHENOL, HC VIOLET no 2, 2-AMINO-6-CHLORO-4-NITROPHENOL, 4-HYDROXYPROPYLAMINO-3-NITROPHENOL, HC YELLOW NO. 13, TETRAHYDRO-6-NITROQUINOXALINE, 2,6-DIAMINO-3-((PYRIDINE-3-YL)AZO)PYRIDINE, BASIC ORANGE 69, HC RED 16 / N-(2-Nitro-4-aminophenyl)-allylamine, BASIC VIOLET 2 als HCl, BASIC RED 51, BASIC YELLOW 87, BASIC ORANGE 31, HC BLUE 16, HC RED No. 17, HC YELLOW 17, HC BLUE 18, HC YELLOW 16, HC RED 18, HC ORANGE 6, BASIC BLUE 124, BASIC RED 76, BASIC BROWN 16, BASIC YELLOW 57, ACID ORANGE 7, ACID RED 33, ACID YELLOW 23, ACID BLUE 9, ACID RED 92, "ACID YELLOW 3 (mono und di sodium)", BASIC BLUE 99, ACID VIOLET 43, DISPERSE VIOLET 1, DISPERSE BLUE 3, ACID BLUE 62, DISPERSE BLACK 9, HYDROXYANTHRAQUINONE AMINOPROPYL METHYL MORPHOLINIUM METHOSULFATE, LAWSONE, LAWSONIA INERMIS, INDIGOFERA TINCTORIA, HC BLUE no 14, CURRY RED, ACID RED 18, ACID RED 52, ACID GREEN 25, DISPERSE BLUE 377, PIGMENT RED 57, HC BLUE No 15, TETRABROMOPHENOL BLUE, HC BLUE No 17 und/oder BISMUTH CITRATE. Bei diesen Farbstoffen sind die Farbstoffe mit der INCI Bezeichnung gemeint. Die Menge an verwendetem Farbstoff hängt sehr stark von der Art des Farbstoffes ab. Als Orientierung für die gewählte Menge dient eine Angabe, dass 0,1 bis 4 Gew.-% Farbstoff in dem Zweikomponentensystem, basierend auf dem Gesamtgewicht des Zweikomponentensystems, enthalten sind.

Das Zweikomponentensystem umfasst gemäß bevorzugter Ausführungsformen Emulgatoren, damit dessen Komponenten, d.h. das wasserfreie Trägermedium und die wässrige Phase, homogenisiert werden können und bei der Herstellung des anwendungsbereiten kosmetischen Mittels eine homogene Phase bilden. Gemäß dieser bevorzugten Ausführungsformen ist der Emulgator oder sind die Emulgatoren entweder in der wässrigen Phase oder in dem wasserfreien Trägermedium enthalten, jedoch bevorzugt in der wässrigen Phase.

Ein bevorzugt verwendeter Emulgator ist eine Alkyltrimoniumverbindung mit einer oder mehreren C8-C22, bevorzugter C10-C18, noch bevorzugter C12-C16 Alkylgruppen. Eine Alkyltrimoniumverbindung ist eine Stickstoff enthaltende Verbindung, die kationisch ist und die mindestens eine, bevorzugt mehrere organische Reste trägt. Die Anzahl der organischen Reste plus Wasserstoff ergibt immer die Zahl vier.

Ein weiterer bevorzugter Emulgator ist ein Fettalkoholethoxylat, bei dem der Fettalkoholteil des Fettalkoholethoxylats eine Alkylkettenlänge von C4-C30, bevorzugt C6-C25, bevorzugter C8-C20, aufweist und/oder bei dem die Anzahl der Ethoxygruppen im Fettalkoholethoxylat bei 2 bis 120, bevorzugt bei 4 bis 100, bevorzugter bei 6 bis 80, noch bevorzugter bei 8 bis 60, am meisten bevorzugt bei 10 bis 40 liegt.

Ferner ist ein bevorzugter Emulgator ein Fettalkoholsulfat mit einer Kettenlänge von C8-C22, bevorzugt von C10-C20, bevorzugter von C12-C18.

Gemäß weiterer bevorzugter Ausführungsformen sind die Emulgatoren in festgelegten Mengen entweder in dem wasserfreien Trägermedium oder in der wässrigen Phase enthalten. Ein bevorzugtes Zweikomponentensystem enthält die Alkyltrimoniumverbindung in einer Menge von 0,1 bis 5 Gew.-%, bevorzugt von 0,5 bis 4 Gew.-%, bevorzugter von 1 bis 3 Gew.-%, bezogen auf das Gesamtgewicht der wässrigen Phase, in der wässrigen Phase, oder die Alkyltrimoniumverbindung in einer Menge von 0,1 bis 5 Gew.-%, bevorzugt von 0,5 bis 4 Gew.-%, bevorzugter von 1 bis 3 Gew.-%, bezogen auf das Gesamtgewicht des wasserfreien Trägermediums, in dem wasserfreien Trägermedium.

Das Fettalkoholethoxylat ist gemäß einer weiteren bevorzugten Ausführungsform in einer Menge von 0,1 bis 10 Gew.-%, bevorzugt von 0,25 bis 7,5 Gew.-%, bevorzugter von 0,5 bis 5 Gew.-%, bezogen auf das Gesamtgewicht der wässrigen Phase, in der wässrigen Phase enthalten, oder das Fettalkoholethoxylat ist in einer Menge von 0,1 bis 10 Gew.-%, bevorzugt von 0,25 bis 7,5 Gew.-%, bevorzugter von 0,5 bis 5 Gew.-%, bezogen auf das Gesamtgewicht des wasserfreien Trägermediums, in dem wasserfreien Trägermedium enthalten.

Ferner ist das Fettalkoholsulfat gemäß bevorzugter Ausführungsformen in einer Menge von 0,1 bis 10 Gew.-%, bevorzugt von 0,25 bis 7,5 Gew.-%, bevorzugter von 0,5 bis 5 Gew.-%, bezogen auf das Gesamtgewicht der wässrigen Phase, in der wässrigen Phase enthalten, oder das Fettalkoholsulfat ist in einer Menge von 0,1 bis 10 Gew.-%, bevorzugt von 0,25 bis 7,5 Gew.-%, bevorzugter von 0,5 bis 5 Gew.-%, bezogen auf das Gesamtgewicht des wasserfreien Trägermediums, in dem wasserfreien Trägermedium enthalten.

Gemäß einer bevorzugten Ausführungsform der vorliegenden Erfindung ist das Wasserstoffperoxid in einer Menge von 1 bis 12 Gew.-%, bevorzugt von 2 bis 10 Gew.-%, bevorzugter von 3 bis 8 Gew.-%, bezogen auf das Gesamtgewicht der wässrigen Phase, in der wässrigen Phase enthalten.

Gemäß einer bevorzugten Ausführungsform der vorliegenden Erfindung beträgt das Gewichtsverhältnis von dem wasserfreien Trägermedium zu der wässrigen Phase bei dem Zweikomponentensystem von 1 zu 10 bis 10 zu 1, bevorzugt 5 zu 1 bis 1 zu 5, bevorzugter von 2 zu 1 bis 1 zu 2.

Grundsätzlich können als Gegenionen der vorliegenden Komponenten, die als Salze vorliegen, alle physiologisch verträgliche Gegenionen verwendet werden.

Die wässrige Phase oder das wasserfreie Trägermedium können in weiteren bevorzugten Ausführungsformen der vorliegenden Erfindung weitere zusätzliche Inhaltsstoffe umfassen. Die folgenden Inhaltsstoffe sind optional und zusätzlich zu den oben beschriebenen Komponenten in dem Zweikomponentensystem enthalten.

In einem weiteren bevorzugten Zweikomponentensystem enthält das wasserfreie Trägermedium oder die wässrige Phase ein kationisches Tensid, wobei das kationische Tensid eines der folgenden Formel ist, 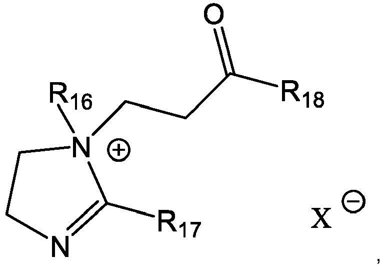 worin
- R₁₆: für eine C1-C6-Alkylgruppe steht
- R₁₇, R₁₈: unabhängig voneinander für eine C7-C27-Alkylgruppe, bevorzugt eine C10-C22-Alkylgruppe stehen und
- X-: für ein physiologisch verträgliches Anion steht,
oder wobei das kationische Tensid eines der folgenden Formel ist, 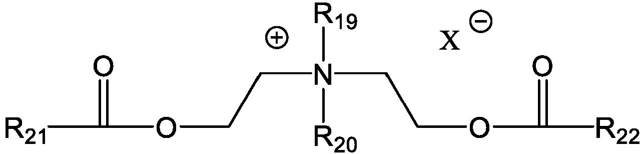 worin
- R₁₉, R₂₀: unabhängig voneinander für eine C1-C6-Alkylgruppe oder eine C2-C6-Hydroxyalkylgruppe stehen,
- R₂₁, R₂₂: unabhängig voneinander für eine C7-C27-Alkylgruppe, bevorzugt eine C10-C22-Alkylgruppe stehen und
- X-: für ein physiologisch verträgliches Anion steht,
oder wobei das kationische Tensid eines der folgenden Formel ist,

NR₂₃R₂₄R₂₅,

worin
- R₂₃, R₂₄: unabhängig voneinander für eine C1-C6-Alkylgruppe, eine C2-C6-Alkenylgruppe oder eine C2-C6-Hydroxyalkylgruppe stehen, und
- R₂₅: für eine C8-C28-Alkylgruppe, bevorzugt eine C10-C22-Alkylgruppe steht.

Bei den kationischen Tensiden der Formel NR₂₃R₂₄R₂₅ handelt es sich um Aminderivate, sogenannte Pseudoquats. Die organischen Reste R₂₃, R₂₄ und R₂₅ sind dabei unmittelbar an das Stickstoffatom gebunden. Im sauren pH-Bereich werden diese kationisiert, d.h. das Stickstoffatom wird dann protoniert. Als Gegenionen bieten sich die physiologisch verträglichen Gegenionen an. Als besonders bevorzugt bietet sich bei diesen kationischen Tensiden Steamidopropyl Dimethylamine an.

Die Tenside unterstützen in der vorliegenden Erfindung der Emulgierung von wässriger Phase und wasserfreiem Trägermedium, bzw. allgemeiner der Emulgierung von Wasser- und Ölphase. Sie sind daher optional zusätzlich in dem Zweikomponentensystem enthalten.

Im Folgenden werden weitere Bestandteile der Haarbehandlungsmittel beschrieben, die neben den zuvor beschriebenen zwingenden Inhaltstoffen in dem wasserfreien Trägermedium oder der wässrigen Phase optional enthalten sein können.

Gemäß weiterer bevorzugter Ausführungsformen enthält das wasserfreie Trägermedium oder die wässrigen Phase ferner ein Hautbefeuchtungsmittel bzw. weiteres Pflegemittel, das ausgewählt ist aus der Gruppe bestehend aus Glycerin, Harnstoff, Hyaluronsäure, Silanolester der Hyaluronsäure, Panthenol, Taurin, Ceramide, Phytosterole, Aloe Vera Extrakte, Kreatin, Kreatinin, Natriumhyaluronat, Polysaccharide, Biosaccharide gum-1, Gurkenextrakte, Butylenglykol, Propylenglykol, Methylpropandiol, Ethylhexylglycerin, Sorbitol, Aminosäuren, wobei Glycin, Glycin Soja, Histidin, Tyrosin oder Tryptophan besonders bevorzugte Aminosäuren sind, Aminosäurederivate, natürliche Betainverbindungen, Pyrrolidoncarbonsäure oder ein Salz der Pyrrolidoncarbonsäure, Milchsäure, Lactate, insbesondere Natriumlactat, und/oder Ethylhexyloxyglycerin. Insbesondere die Auswahl dieser Hautbefeuchtungsmittel erhöhen den pflegenden Charakter des wasserfreien Trägermediums.

Gemäß einer weiteren bevorzugten Ausführungsform enthält das wasserfreie Trägermedium oder die wässrige Phase mehrere Emulgatoren oder mehrere Tenside. Es ist insbesondere bevorzugt, dass das wasserfreie Trägermedium oder die wässrige Phase zwei strukturell voneinander verschiedene Tenside/Emulgatoren enthält, wobei bevorzugt das wasserfreie Trägermedium oder die wässrige Phase zwei strukturell voneinander verschiedene kationische Emulgatoren/Tenside, zwei voneinander verschiedene anionische Emulgatoren/Tenside, ein(en) kationisches/n Tensid/Emulgator und ein(en) nichtionisches/N Tensid/Emulgator, oder ein(en) anionisches/n Tensid/Emulgator und ein(en) nichtionisches/n Tensid/Emulgator enthält.

Gemäß einer bevorzugten Ausführungsform der vorliegenden Erfindung umfasst das eingesetzte kationische Tensid eine hydrophobe Kopfgruppe mit einer kationischen Ladung und einen oder zwei hydrophobe Endteile, wobei das hydrophobe Endteil oder die hydrophoben Endteile geradkettige oder verzweigte, gesättigte oder ein- oder mehrfach ungesättigte Alkylgruppen darstellen, die bevorzugt eine Kettenlänge von C6 bis C30, bevorzugter C8 bis C26, besonders bevorzugt C10 bis C22, aufweisen.

Gemäß einer weiteren bevorzugten Ausführungsform weist das kationische Tensid eine Esterfunktion, eine Etherfunktion, eine Ketonfunktion, eine Alkoholfunktion oder eine Amidfunktion auf.

Gemäß einer bevorzugten Ausführungsform der vorliegenden Erfindung enthält das wasserfreie Trägermedium oder die wässrige Phase als eine weitere Komponente ein weiteres nichtionisches Tensid, das bevorzugt ein nichtionisches Tensid ausgewählt aus der Gruppe bestehend aus den folgenden umfasst:
- Alkylglucamid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylfructosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylglucosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe, und
- Alkylalkoholalkoxylat der Formel R₁₀(OR₁₁)ₘOH, in der R₁₀ eine lineare oder verzweigte C₆ bis C₂₂, bevorzugt C₁₀ bis C₁₈, bevorzugter C₁₂ bis C₁₆ Alkylgruppe, R₁₁ eine C₂ bis C₄, bevorzugt eine C₂ Alkylgruppe, und m 1 bis 10, bevorzugt 2 bis 6, bevorzugter 2 bis 6, darstellen.

Gemäß bevorzugter Ausführungsformen der vorliegenden Erfindung sind ein oder mehrere weitere anionische Tenside als eine Komponente in dem wasserfreien Trägermedium oder der wässrigen Phase enthalten, die bevorzugt ausgewählt ist aus der Gruppe bestehend aus
- linearen Alpha-Olefinsulfonaten mit 8 bis 24, bevorzugt 12 bis 22, bevorzugter 16 bis 18 C-Atomen,
- geradkettigen oder verzweigten, gesättigten oder ein- oder mehrfach ungesättigten Alkylcarbonsäuren mit 8 bis 24, bevorzugt 12 bis 22, bevorzugter 16 bis 18 C-Atomen,
- geradkettigen oder verzweigten, gesättigten oder ein- oder mehrfach ungesättigten Alkylphosphaten mit 8 bis 24, bevorzugt 12 bis 22, bevorzugter 16 bis 18 C-Atomen,
- Alkylisethionat, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe, insbesondere Natriumcocoylisethionat,
- Alkylglycosidcarbonsäuren, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Alkylsulfosuccinaten, dessen zwei Alkylgruppen ausgewählt sind aus gleichen oder verschiedenen, verzweigte oder unverzweigten C₂ bis C₁₂, bevorzugt C₄ bis C₁₀, bevorzugter C₆ bis C₈ Alkylgruppen,
- Alkyltauraten, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Alkylsarcosinaten, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Sulfonate ungesättigter Fettsäuren mit 8 bis 24, bevorzugt 12 bis 22, bevorzugter 16 bis 18 C-Atomen und 1 bis 6 Doppelbindungen,
wobei das Gegenion des anionischen Tensids ein Alkali- oder Erdalkalimetallion oder ein protoniertes Triethanolamin oder das Ammonium-Ion ist.

Ganz besonders bevorzugt enthält wässrige Phase eine Tensidmischung aus anionischen und amphoteren/zwitterionischen Tensiden Natriumlaurylethersulfat (INCI: Sodium Laureth Sulfate) und ganz besonders bevorzugt Natriumlaurylethersulfat mit 2 Ethylenoxideinheiten.

Amphotere Tenside, welche auch als zwitterionische Tenside bezeichnet werden, werden solche oberflächenaktiven Verbindungen genannt, die im Molekül mindestens eine quartäre Ammoniumgruppe und mindestens eine -COO⁻ - oder -SO₃⁻ -Gruppe tragen. Unter amphoteren/zwitterionischen Tensiden werden auch solche oberflächenaktiven Verbindungen verstanden, die außer einer C₈ - C₂₄ -Alkyl- oder - Acylgruppe mindestens eine freie Aminogruppe und mindestens eine -COOH- oder -SO₃H-Gruppe enthalten und zur Ausbildung innerer Salze befähigt sind.

Gemäß einer bevorzugten Ausführungsform der vorliegenden Erfindung enthält das wasserfreie Trägermedium oder die wässrige Phase als eine weitere Komponente mindestens ein amphoteres Tensid. Bevorzugt sind die amphoteren Tenside in dem wasserfreie Trägermedium ausgewählt aus der Gruppe bestehend aus
- Alkylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylamphodiacetat oder Alkylamphodiacetat, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe, mit einem Alkali- oder Erdalkalimetallgegenion, und
- Alkylamidopropylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe.

Zu den insbesondere geeigneten amphoteren/zwitterionischen Tensiden zählen die unter der INCI-Bezeichnung bekannten Tenside Cocamidopropylbetain und Disodium Cocoamphodiacetate.

Das wasserfreie Trägermedium kann insbesondere verwendet werden zur Herstellung eines Mittels zur Färbung eines keratinischen Materials, eines Mittels zur Pflege eines keratinischen Materials und/oder eines Mittels zur Pflege und Färbung eines keratinischen Materials.

Es kann bevorzugt sein, dass das wasserfreie Trägermedium oder die wässrige Phase zur Behandlung eines keratinischen Materials ferner 0,001 bis 20 Gew.-% mindestens einer quaternären Verbindung umfasst. Dies gilt insbesondere für wasserfreie Trägermedien oder wässrigen Phasen, die zur Herstellung eines Mittels zur Pflege eines keratinischen Materials oder zur Pflege und Reinigung eines keratinischen Materials verwendet werden.

Es ist bevorzugt, dass die mindestens eine quaternäre Verbindung ausgewählt aus mindestens einer der Gruppen bestehend aus
i) der Monoalkylquats und/oder
ii) der Esterquats und/oder
iii) der quaternären Imidazoline der Formel (Tkat2), 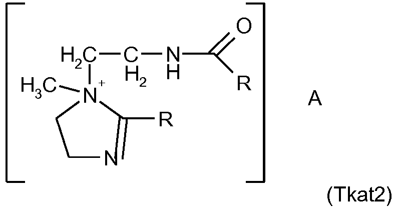 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
iv) der Amidoamine und/oder kationisierten Amidoamine und/oder
v) Poly(methacryloyloxyethyltrimethylammoniumverbindungen) und/oder;
vi) quaternisierten Cellulose-Derivaten, insbesondere Polyquaternium 10, Polyquaternium-24, Polyquaternium-27, Polyquaternium-67, Polyquaternium-72, und/oder
vii) kationischen Alkylpolyglycosiden und/oder
viii) kationisiertem Honig und/oder
ix) kationischen Guar-Derivaten und/oder
x) Chitosan und/oder
xi) polymeren Dimethyldiallylammoniumsalzen und deren Copolymeren mit Estern und Amiden von Acrylsäure und Methacrylsäure, insbesondere Polyquaternium-7 und/oder
xii) Copolymeren des Vinylpyrrolidons mit quaternierten Derivaten des Dialkylaminoalkylacrylats und -methacrylats, insbesondere Polyquaternium-11 und/oder
xiii) Vinylpyrrolidon-Vinylimidazoliummethochlorid-Copolymeren, insbesondere Polyquaternium-16 und/oder
xiv) quaterniertem Polyvinylalkohol und/oder
xv) Polyquaternium-74,
sowie Mischungen hiervon.

Es ist insbesondere bevorzugt, dass das wasserfreie Trägermedium oder die wässrige Phase ein kationisches Homopolymer, welches unter die INCI-Bezeichnung Polyquaternium-37 fällt, als quaternäre Verbindungen enthält.

Es kann bevorzugt sein, dass das wasserfreie Trägermedium oder die wässrige Phase ferner eine festigende Verbindung, vorzugsweise ausgewählt aus der Gruppe bestehend aus Wachsen, synthetischen Polymeren und Mischungen daraus, umfasst.

Um den unterschiedlichen Anforderungen an kosmetischen Mitteln gerecht zu werden, die zur Herstellung eines Mittels zur Behandlung eines keratinischen Materials zum temporären Umformen eines keratinischen Materials (= Stylingmittel) verwendet werden, sind als festigende Verbindungen bereits eine Vielzahl von synthetischen Polymeren entwickelt worden, die in dem Mittel zur Behandlung eines keratinischen Materials zur Anwendung kommen können. Alternativ oder ergänzend werden Wachse als festigende Verbindungen eingesetzt. Idealerweise ergeben die Polymere und/oder Wachse bei der Anwendung auf dem keratinischen Material einen Polymerfilm oder Film, der einerseits der Frisur einen starken Halt verleiht, andererseits aber hinreichend flexibel ist, um bei Beanspruchung nicht zu brechen.

Die synthetischen Polymere lassen sich in kationische, anionische, nichtionische und amphotere festigende Polymere unterteilen.

Geeignete synthetische Polymere umfassen beispielsweise Polymere mit den folgenden INCI-Bezeichnungen: Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/Hydroxyesters Acrylates Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis- Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer. Ebenso geeinet sind Celluloseether, wie Hydroxypropylcellulose, Hydroxyethylcellulose und Methylhydroxypropylcellulose.

Auch Homopolyacrylsäure (INCI: Carbomer), die im Handel unter dem Namen Carbopol^{®} in unterschiedlichen Ausführungen erhältlich ist, ist als festigende Verbindung geeignet.

Bevorzugt umfasst die festigende Verbindung ein Vinylpyrrolidon-haltiges Polymer. Besonders bevorzugt umfasst die festigende Verbindung ein Polymer ausgewählt aus der Gruppe bestehend aus Polyvinylpyrrolidon (PVP), Vinylpyrrolidon-Vinylacetat-Copolymer (VP/VA Coplymer), Vinyl Caprolactam/VP/Dimethylaminoethyl Methacrylate Copolymer (INCI), VP/DMAPA Acrylates Copolymer (INCI) und Mischungen daraus.

Eine ebenfalls bevorzugte festigende Verbindung ist Octylacrylamide/Acrylates/Butylaminoethyl Methacrylate Copolymer (INCI), welches unter der Bezeichnung "Amphomer^{®}" von Akzo Nobel vertrieben wird.

Entsprechend ist es besonders bevorzugt, dass die festigende Verbindung ein synthetisches Polymer ausgewählt aus der Gruppe bestehend aus Polyvinylpyrrolidon (PVP), Vinylpyrrolidon-Vinylacetat-Copolymer (VP/VA Coplymer), Vinyl Caprolactam/VP/Dimethylaminoethyl Methacrylate Copolymer (INCI), VP/DMAPA Acrylates Copolymer (INCI), Octylacrylamide/Acrylates/Butylaminoethyl Methacrylate Copolymer (INCI) und Mischungen daraus umfasst.

Die kosmetischen Mittel können zusätzlich oder alternativ zu einem synthetischen Polymer mindestens ein natürliches oder synthetisches Wachs, welches einen Schmelzpunkt von über 37 °C aufweist, als festigende Verbindung enthalten.

Als natürliche oder synthetische Wachse können feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie zum Beispiel Bienenwachse und andere Insektenwachse, Walrat, Schellackwachs, Wollwachs und Bürzelfett, weiterhin Mineralwachse, wie zum Beispiel Ceresin und Ozokerit oder die petrochemischen Wachse, wie zum Beispiel Petrolatum, Paraffinwachse, Microwachse aus Polyethylen oder Polypropylen und Polyethylenglycolwachse eingesetzt werden. Es kann vorteilhaft sein, hydrierte oder gehärtete Wachse einzusetzen. Weiterhin sind auch chemisch modifizierte Wachse, insbesondere die Hartwachse, zum Beispiel Montanesterwachse, Sasolwachse und hydrierte Jojobawachse, einsetzbar.

Weiterhin geeignet sind die Triglyceride gesättigter und gegebenenfalls hydroxylierter C16-30-Fettsäuren, wie zum Beispiel gehärtete Triglyceridfette (hydriertes Palmöl, hydriertes Kokosöl, hydriertes Rizinusöl), Glyceryltribehenat oder Glyceryltri-12-hydroxystearat.

Die Wachskomponenten können auch aus der Gruppe der Ester aus gesättigten, unverzweigten Alkancarbonsäuren einer Kettenlänge von 22 bis 44 C-Atomen und gesättigten, unverzweigten Alkoholen einer Kettenlänge von 22 bis 44 C-Atomen ausgewählt werden, sofern die Wachskomponente oder die Gesamtheit der Wachskomponenten bei Raumtemperatur fest sind. Auch Silikonwachse, zum Beispiel Stearyltrimethylsilan/Stearylalkohol sind gegebenenfalls vorteilhaft.

Natürliche, chemisch modifizierte und synthetische Wachse können alleine oder in Kombination eingesetzt werden. Dazu sollen jedoch keine Alkane gezählt werden, die in dem erfindungsgemäßen wasserfreien Trägermedium zwingend enthalten sind. Es können auch mehrere Wachse eingesetzt werden. Weiterhin ist auch eine Reihe von Wachsmischungen, ggf. in Abmischung mit weiteren Zusätzen, im Handel erhältlich. Die unter den Bezeichnungen "Spezialwachs 7686 OE" (eine Mischung aus Cetylpalmitat, Bienenwachs, mikrokristallinem Wachs und Polyethylen mit einem Schmelzbereich von 73-75 °C; Hersteller: Kahl & Co), Polywax^{®} GP 200 (eine Mischung von Stearylalkohol und Polyethylenglykolstearat mit einem Schmelzpunkt von 47-51 °C; Hersteller: Croda) und "Weichceresin^{®} FL 400" (ein Vaseline/Vaselinöl/Wachs-Gemisch mit einem Schmelzpunkt von 50-54 °C; Hersteller: Parafluid Mineralölgesellschaft) sind Beispiele für einsetzbare Mischungen.

Bevorzugt ist das Wachs ausgewählt aus Carnaubawachs (INCI: Copernicia Cerifera Cera) Bienenwachs (INCI: Beeswax), Petrolatum (INCI), mikrokristallinem Wachs und insbesondere Gemischen daraus.

Bevorzugte Mischungen umfassen die Kombination von Carnaubawachs (INCI: Copernicia Cerifera Cera), Petrolatum und mikrokristallinem Wachs oder die Kombination von Bienenwachs (INCI: Beeswax) und Petrolatum.

Das Wachs oder die Wachskomponenten sollten bei 25 °C fest sein und sollen im Bereich von > 37 °C schmelzen

Das wasserfreie Trägermedium oder die wässrige Phase enthält die festigende Verbindung vorzugsweise in einer Gesamtmenge von 0,5 bis 50 Gew.-%, bevorzugt 1 bis 40 Gew.-%, weiter bevorzugt 1,5 bis 30 Gew.-%, noch mehr bevorzugt 2 bis 25 Gew.-%, bezogen auf das Gesamtgewicht der kosmetischen Zusammensetzung.

Weitere geeignete Inhaltsstoffe umfassen nichtionische Polymere, anionische Polymere, (weitere) kationische Polymere, Wachse, Proteinhydrolysate, Aminosäuren, Oligopetide, Vitamine, Provitamine, Vitaminvorstufen, Betaine, Biochinone, Purin(derivate), Pflanzenextrakte, Silikone, Esteröle, UV-Lichtschutzfilter, Strukturierungsmittel, Verdickungsmittel, Elektrolyte, pH-Stellmittel, Quellmittel, Farbstoffe, Antischuppenwirkstoffe, Komplexbildner, Trübungsmittel, Perlglanzmittel, Pigmente, Stabilisierungsmittel, Treibmittel, Antioxidantien, Parfümöle und/oder Konservierungsmittel.

Ein weiterer Gegenstand der vorliegenden Anmeldung ist die Verwendung des Zweikomponentensystems zur Färbung eines keratinischen Materials oder zur Pflege und Färbung eines keratinischen Materials.

Bezüglich weiterer bevorzugter Ausführungsformen der Verwendung gilt mutatis mutandis das zu den wasserfreien Trägermedien Gesagte.

## Patentansprüche

1. Zweikomponentensystem zur künstlichen Färbung von keratinischem Material, getrennt voneinander umfassend
• ein wasserfreies Trägermedium als erste Komponente, umfassend
a) mindestens ein verzweigtes oder lineares C8-C30 Alkan,
b) mindestens einen verzweigten oder linearen C10-C30 Fettalkohol,
c) mindestens einen verzweigten oder linearen einwertigen C2-C8 Alkohol,
d) mindestens ein verzweigtes oder lineares C2-C10 Amin als Alkalisierungsmittel,
e) einen oder mehrere Farbstoffe zur Farbänderung des keratinischen Materials, und
f) mindestens eine organische Siliciumverbindung, wobei die mindestens eine organische Siliciumverbindung eine Verbindung der Formel (I) und mindestens eine organische Siliciumverbindung der Formel (IV) enthält,
wobei in der organischen Siliciumverbindung der Formel (I)
R₁R₂N-L-Si(OR₃)ₐ(R₄)_{b} (I),
- R₁, R₂ beide für ein Wasserstoffatom stehen,
- L für eine lineare, zweibindige C₁-C₆-Alkylengruppe, bevorzugt für eine Propylengruppe
(-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und
- b für die Zahl 0 steht, und
wobei in der organischen Siliciumverbindung der Formel (IV)
R₉Si(OR₁₀)ₖ(R₁₁)ₘ (IV),
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht, und
• eine wässrige Phase als zweite Komponente, umfassend Wasser und Wasserstoffperoxid.

2. Zweikomponentensystem gemäß Anspruch 1, **dadurch gekennzeichnet, dass** das wasserfreie Trägermedium ferner mindestens eine organische Siliciumverbindung umfasst, die eine Verbindung der Formel (II) darstellt,
wobei in der organischen Siliciumverbindung der Formel (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} und R_{6"} unabhängig voneinander für eine C₁-C₆-Alkylgruppe stehen,
- A, A', A", A‴ und A"" unabhängig voneinander für eine lineare oder verzweigte, zweibindige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III) stehen
- (Aʺʺ)-Si(Rₑ")_{d}"(OR₅")_{c}" (III),
- c für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
mit der Maßgabe, dass mindestens einer der Reste aus e, f, g und h von 0 verschieden ist.

3. Zweikomponentensystem gemäß einem der Ansprüche 1 oder 2,
**dadurch gekennzeichnet,**
**dass** das Zweikomponentensystem zur Behandlung eines keratinischen Materials mindestens eine organische Siliciumverbindung der Formel (I) enthält, die ausgewählt ist aus der Gruppe bestehend aus
- (3-Aminopropyl)trimethoxysilan
- (3-Aminopropyl)triethoxysilan
- (2-Aminoethyl)trimethoxysilan und
- (2-Aminoethyl)triethoxysilan.

4. Zweikomponentensystem gemäß Anspruch 2 oder 3, **dadurch gekennzeichnet,**
**dass** das Zweikomponentensystem mindestens eine organische Siliciumverbindung der Formel (II) enthält, die ausgewählt ist aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und
- N,N-Bis[3-(triethoxysilyl)propyl]-2-Propen-1-amin.

5. Zweikomponentensystem gemäß einem der Ansprüche 1 bis 4, **dadurch gekennzeichnet, dass** die organische Siliciumverbindung in einer Menge von 0,01 bis 10 Gew.-%, bevorzugt von 0,02 bis 9 Gew.-%, bevorzugter von 0,05 bis 8 Gew.-%, am meisten bevorzugt von 0,1 bis 7 Gew.-%, bezogen auf das Gesamtgewicht des wasserfreien Trägermediums, in dem wasserfreien Trägermedium enthalten ist, wobei die organische Siliciumverbindung insbesondere (3-Aminopropyl)triethoxysilan ist.

6. Zweikomponentensystem gemäß einem der Ansprüche 1 bis 5, **dadurch gekennzeichnet, dass** das Zweikomponentensystem mindestens eine organische Siliciumverbindung der Formel (IV) enthält,
R₉Si(OR₁₀)ₖ(R₁₁)ₘ (IV),
die ausgewählt ist aus der Gruppe bestehend aus
- Methyltrimethoxysilan
- Methyltriethoxysilan
- Ethyltrimethoxysilan
- Ethyltriethoxysilan
- Propyltrimethoxysilan
- Propyltriethoxysilan
- Hexyltrimethoxysilan
- Hexyltriethoxysilan
- Octyltrimethoxysilan
- Octyltriethoxysilan
- Dodecyltrimethoxysilan und
- Dodecyltriethoxysilan.

7. Zweikomponentensystem gemäß einem der Ansprüche 1 bis 6, **dadurch gekennzeichnet, dass** das Alkan ein C10-C24 Alkan, bevorzugt ein C12-C18 Alkan und bevorzugter ein C14-C16 Alkan ist, und/oder wobei das Alkan in einer Menge von 1 bis 50 Gew.-%, bevorzugt 2 bis 45 Gew.-%, bevorzugter 3 bis 40 Gew.-%, noch bevorzugter 4 bis 35 Gew.-%, bezogen auf das Gesamtgewicht des wasserfreien Trägermediums, in dem wasserfreien Trägermedium enthalten ist, und/oder **dadurch gekennzeichnet, dass**
der Fettalkohol ein C12-C24 Fettalkohol, bevorzugt ein C14-C18 Fettalkohol ist, und/oder wobei der Fettalkohol in einer Menge von 5 bis 50 Gew.-%, bevorzugt 6 bis 45 Gew.-%, bevorzugter 7 bis 40 Gew.-%, noch bevorzugter 8 bis 35 Gew.-%, bezogen auf das Gesamtgewicht des wasserfreien Trägermediums, in dem wasserfreien Trägermedium enthalten ist, und/oder **dadurch gekennzeichnet, dass**
der Alkohol ein C3-C6 Alkohol, bevorzugt ein C4-C5 Alkohol ist, und/oder wobei der Alkohol in einer Menge von 4 bis 50 Gew.-%, bevorzugt 6 bis 45 Gew.-%, bevorzugter 8 bis 40 Gew.-%, noch bevorzugter 10 bis 30 Gew.-%, bezogen auf das Gesamtgewicht des wasserfreien Trägermediums, in dem wasserfreien Trägermedium enthalten ist und/oder **dadurch gekennzeichnet, dass**
das verzweigte oder lineare Amin ein C3-C8, bevorzugter ein C4-C6 Amin ist, und/oder wobei das Amin in einer Menge von 0,5 bis 10 Gew.-%, bevorzugt von 0,75 bis 7,5 Gew.-%, bevorzugter von 1 bis 5 Gew.-%, bezogen auf das Gesamtgewicht des wasserfreien Trägers, im wasserfreien Träger enthalten ist.

8. Zweikomponentensystem gemäß einem der Ansprüche 1 bis 7, **dadurch gekennzeichnet, dass** die wässrige Phase oder das wasserfreie Trägermedium einen oder mehrere Emulgatoren umfasst,
wobei der Emulgator bevorzugt eine Alkyltrimoniumverbindung mit einer oder mehreren C8-C22, bevorzugter C10-C18, noch bevorzugter C12-C16 Alkylgruppen ist, und/oder wobei der Emulgator bevorzugt ein Fettalkoholethoxylat ist, bei dem der Fettalkoholteil des Fettalkoholethoxylats eine Alkylkettenlänge von C4-C30, bevorzugt C6-C25, bevorzugter C8-C20, aufweist und/oder bei dem die Anzahl der Ethoxygruppen im Fettalkoholethoxylat bei 2 bis 120, bevorzugt bei 4 bis 100, bevorzugter bei 6 bis 80, noch bevorzugter bei 8 bis 60, am meisten bevorzugt bei 10 bis 40 liegt, und/oder
wobei der Emulgator bevorzugt ein Fettalkoholsulfat mit einer Kettenlänge von C8-C22, bevorzugt von C10-C20, bevorzugter von C12-C18 ist.

9. Zweikomponentensystem gemäß einem der Ansprüche 1 bis 8, **dadurch gekennzeichnet, dass** das die Alkyltrimoniumverbindung in einer Menge von 0,1 bis 5 Gew.-%, bevorzugt von 0,5 bis 4 Gew.-%, bevorzugter von 1 bis 3 Gew.-%, bezogen auf das Gesamtgewicht der wässrigen Phase, in der wässrigen Phase enthalten ist, oder die Alkyltrimoniumverbindung in einer Menge von 0,1 bis 5 Gew.-%, bevorzugt von 0,5 bis 4 Gew.-%, bevorzugter von 1 bis 3 Gew.-%, bezogen auf das Gesamtgewicht des wasserfreien Trägermediums, in dem wasserfreien Trägermedium enthalten ist, und/oder **dadurch gekennzeichnet, dass** das Fettalkoholethoxylat in einer Menge von 0,1 bis 10 Gew.-%, bevorzugt von 0,25 bis 7,5 Gew.-%, bevorzugter von 0,5 bis 5 Gew.-%, bezogen auf das Gesamtgewicht der wässrigen Phase, in der wässrigen Phase enthalten ist, oder das Fettalkoholethoxylat in einer Menge von 0,1 bis 10 Gew.-%, bevorzugt von 0,25 bis 7,5 Gew.-%, bevorzugter von 0,5 bis 5 Gew.-%, bezogen auf das Gesamtgewicht des wasserfreien Trägermediums, in dem wasserfreien Trägermedium enthalten ist, und/oder **dadurch gekennzeichnet, dass** das Fettalkoholsulfat in einer Menge von 0,1 bis 10 Gew.-%, bevorzugt von 0,25 bis 7,5 Gew.-%, bevorzugter von 0,5 bis 5 Gew.-%, bezogen auf das Gesamtgewicht der wässrigen Phase, in der wässrigen Phase enthalten ist, oder das Fettalkoholsulfat in einer Menge von 0,1 bis 10 Gew.-%, bevorzugt von 0,25 bis 7,5 Gew.-%, bevorzugter von 0,5 bis 5 Gew.-%, bezogen auf das Gesamtgewicht des wasserfreien Trägermediums, in dem wasserfreien Trägermedium enthalten ist.

10. Zweikomponentensystem gemäß einem der Ansprüche 1 bis 9, **dadurch gekennzeichnet, dass** das Wasserstoffperoxid in einer Menge von 1 bis 12 Gew.-%, bevorzugt von 2 bis 10 Gew.-%, bevorzugter von 3 bis 8 Gew.-%, bezogen auf das Gesamtgewicht der wässrigen Phase, in der wässrigen Phase enthalten ist, und/oder **dadurch gekennzeichnet, dass**
der Farbstoff zur Farbveränderung ausgewählt ist aus der Gruppe bestehend aus
1,7-NAPHTHALENEDIOL, m-PHENYLENEDIAMINE, TOLUENE-2,5-DIAMINE SULFATE, 2,4-DIAMINOANISOL, p-PHENYLENEDIAMINE HCl, p-PHENYLENEDIAMINE (free base), 2-CHLORO-p-PHENYLENEDIAMINE, N-PHENYL-p-PHENYLENEDIAMINE, RESORCINOL, 4-CHLORORESORCINOL, o-AMINOPHENOL, m-AMINOPHENOL, p-AMINOPHENOL, 1-NAPHTHOL, 1,5-NAPHTHALENEDIOL, 2,7-NAPHTHALENEDIOL, HYDROQUINONE, p-METHYLAMINOPHENOL, p-METHYLAMINOPHENOLSULFATE, HYDROXYBENZOMORPHOLINE, 4-AMINO-2-HYDROXYTOLUENE, 2-METHYL-5-HYDROXYETHYLAMINOPHENOL, 1,2,4-TRIHYDROXYBENZENE, PHENYL METHYL PYRAZOLONE, 2,4-DIAMINOPHENOXYETHANOL HCl oder SO4, 3-AMINO-2,4-DICHLOROPHENOL HCl, 2-METHYLRESORCINOL, N,N-BIS(2-HYDROXYETHYL)-p-PHENYLENEDIAMINE SULFATE, TETRAAMINOPYRIMIDINE SULFATE, 4-AMINO-m-CRESOL, 6-AMINO-m-CRESOL, 1,3-BIS-(2,4-DIAMINOPHENOXY)PROPANE x 4 HCl, HYDROXYETHYL-p-PHENYLENEDIAMINE SULFATE, 2-AMINO-4-HYDROXYETHYLAMINOANISOLE SULFATE, 4,4-DIAMINODIPHENYLAMINE SULFATE, 5-AMINO-6-CHLORO-o-CRESOL, HYDROXYETHYL-3,4-METHYLENEDIOXYANILINE HCl, 2,6-DIHYDROXY-3,4-DIMETHYLPYRIDINE, 2,6-DIMETHOXY-3,5-PYRIDINEDIAMIE x (2) HCl, DIHYDROXYINDOLE, 2-AMINOMETHYL-p-AMINOPHENOL HCl, 2,4-DIAMINO-5-METHYLPHENETOL HCl, 5-AMINO-4-CHLORO-o-CRESOL HCl, HYDROXYPROPYL BIS( N-HYDROXYETHYL-p-PHENYLENEDIMAMINE) HCl, 6-HYDROXYINDOLE, ISATIN, 6-METOXY-2-METHYLAMINO-3-AMINOPYRIDINE HCl (HC BLUE 7), 2-AMINO-3-HYDROXYPYRIDINE, 2-DIMETHYLAMINO-5-AMINOPYRIDINE x2HCL, 6-METHOXY-2,3-PYRIDINEDIAMINE HCL, 2,6-DIAMINO PYRIDINE, 2,6-DIHYDROXYETHYLAMINOTOLUENE, 2,5,6-TRIAMINO-4_PYRIMIDINOL SULFATE, DIHYDROXYINDOLINE HBr, 1-ACETOXY-2-METHYLNAPHTHALENE, 1-HYDROXYETHYL 4,5-DIAMINOPYRAZOLE SULFATE ,2,2'-METHYLENEBIS 4-AMINOPHENOL HCl, 2-METHYL-1-NAPHTHOL, 4-Formyl 1-methylchinolinium-p-Toluene sulfonate, 2-AMINO-5-ETHYLPHENYL HCL, 2,3-DIAMINODIHYDROXY PYRAZOLOPYRAZOLONE DIMETHOSULFONATE, 2-METHOXY-METHYL-p-PHENYLENEDIAMINE, HYDROXYETHOXY AMINOPYRAZOLOPYRIDINE HCL, 3-AMINO-2,6-DIMETHYLPHENOL, 1-HEXYL 4,5-DIAMINO PYRAZOLE, SULFATE, DIMETHYLPIPERAZINIUM AMINOPYRAZOLO PYRIDINE, METHYLIMIDAZOLIUM p-PHENYLENEDIAMINE, ACID YELLOW 1, DISPERSE RED 17,BASIC BROWN 17, ACID BLACK 52, ACID BLACK 1, DISPERSE VIOLET 4, 4-NITRO-o-PHENYLENDIAMINE, 2-NITRO-P-PHENYLENEDIAMINE, 2-AMINO-4-NITROPHENOL, 2-AMINO-5-NITROPHENOL, PICRAMIC ACID and SODIUM PICRAMATE, HC RED NO. 13, N,N'-BIS(2-HYDROXYETHYL)-2-NITRO-p-PHENYLENEDIAMINE, HC YELLOW No 5, HC RED NO. 7, HC BLUE NO. 2, HC YELLOW NO. 4, HC YELLOW NO. 2, HC ORANGE NO. 1, HC RED NO. 1, HC RED NO. 3, 4-AMINO-3-NITROPHENOL, 2-HYDROXYETHYLAMINO-5-NITROANISOLE, 3-NITRO-p-HYDROXYETHYLAMINOPHENOL, 3-METHYLAMINO-4-NITROPHENOXYETHANOL, 2-NITRO-5-GLYCERYL METHYLANILINE, HC VIOLET no 1, HC ORANGE no 2, HC YELLOW No 9, 4-NITROPHENYLAMINOETHYLUREA, HC RED NO. 10 + HC RED NO. 11, 2-HYDROXYETHYL PICRAMIC ACID, HC BLUE NO. 12 als HCl, HC YELLOW NO. 6, HYDROXYETHYL-2-NITRO-p-TOLUIDINE, HC YELLOW NO. 12, HC BLUE No 11, HC YELLOW no 7, HC YELLOW no 10, 4-AMINO-2-NITRO-DIPHENYL-AMINE-2'-CARBOXYLIC ACID, 2-CHLORO-6-ETHYLAMINO-4-NITROPHENOL, HC VIOLET no 2, 2-AMINO-6-CHLORO-4-NITROPHENOL, 4-HYDROXYPROPYLAMINO-3-NITROPHENOL, HC YELLOW NO. 13, TETRAHYDRO-6-NITROQUINOXALINE, 2,6-DIAMINO-3-((PYRIDINE-3-YL)AZO)PYRIDINE, BASIC ORANGE 69, HC RED 16 / N-(2-Nitro-4-aminophenyl)-allylamine, BASIC VIOLET 2 als HCl, BASIC RED 51, BASIC YELLOW 87, BASIC ORANGE 31, HC BLUE 16, HC RED No. 17, HC YELLOW 17, HC BLUE 18, HC YELLOW 16, HC RED 18, HC ORANGE 6, BASIC BLUE 124, BASIC RED 76, BASIC BROWN 16, BASIC YELLOW 57, ACID ORANGE 7, ACID RED 33, ACID YELLOW 23, ACID BLUE 9, ACID RED 92, "ACID YELLOW 3 (mono und di sodium)", BASIC BLUE 99, ACID VIOLET 43, DISPERSE VIOLET 1, DISPERSE BLUE 3, ACID BLUE 62, DISPERSE BLACK 9, HYDROXYANTHRAQUINONE AMINOPROPYL METHYL MORPHOLINIUM METHOSULFATE, LAWSONE, LAWSONIA INERMIS, INDIGOFERA TINCTORIA, HC BLUE no 14, CURRY RED, ACID RED 18, ACID RED 52, ACID GREEN 25, DISPERSE BLUE 377, PIGMENT RED 57, HC BLUE No 15, TETRABROMOPHENOL BLUE, HC BLUE No 17 oder BISMUTH CITRATE.

11. Zweikomponentensystem gemäß einem der Ansprüche 1 bis 10, **dadurch gekennzeichnet, dass** das Gewichtsverhältnis von dem wasserfreien Trägermedium zu der wässrigen Phase im Bereich von 1 zu 10 bis 10 zu 1, bevorzugt 5 zu 1 bis 1 zu 5, bevorzugter von 2 zu 1 bis 1 zu 2 liegt.

12. Verwendung eines Zweikomponentensystems gemäß einem der Ansprüche 1 bis 11 zur Färbung eines keratinischen Materials oder zur Pflege und Färbung eines keratinischen Materials.

## Claims

1. A two-component system for artificially coloring keratinous material, separately comprising
• a water-free carrier medium as a first component, comprising
a) at least one branched or linear C8-C30 alkane,
b) at least one branched or linear C10-C30 fatty alcohol,
c) at least one branched or linear monohydric C2-C8 alcohol,
d) at least one branched or linear C2-C10 amine as an alkalizing agent,
e) one or more dyes for changing the color of the keratinous material, and
f) at least one organosilicon compound, wherein the at least one organosilicon compound contains a compound of formula (I) and at least one organosilicon compound of formula (IV),
where, in the organosilicon compound of formula (I),
R₁R₂N-L-Si(OR₃)ₐ(R₄)_{b} (I),
- R₁ and R2 both represent a hydrogen atom,
- L represents a linear, divalent C₁-C ₆-alkylene group, preferably a propylene group
(-CH₂-CH₂-CH₂-) or an ethylene group (-CH₂-CH₂-),
- R₃ and R₄ independently represent a methyl group or an ethyl group,
- a represents the number 3, and
- b represents the number 0, and
where, in the organosilicon compound of formula (IV)
R₉Si(OR₁₀)ₖ(R₁₁)ₘ (IV),
- R₉ represents a C₁-C₁₂ alkyl group,
- R₁₀ represents a hydrogen atom or a C1-C₆ alkyl group,
- R₁₁ represents a C₁-C₆ alkyl group,
- k represents an integer from 1 to 3, and
- m represents the integer 3 - k, and
• an aqueous phase as a second component, comprising water and hydrogen peroxide.

2. The two-component system according to claim 1, **characterized in that** the water-free carrier medium further comprises at least one organosilicon compound which is a compound of formula (II),
where, in the organosilicon compound of formula (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- Rs, R_{5'}, R_{5"} R₆, R_{6'} and R_{6"} independently represent a C₁-C₆ alkyl group,
- A, A', A", A‴ and Aʺʺ independently represent a linear or branched, divalent C₁-C20 alkylene group,
- R₇ and R₈ independently represent a hydrogen atom, a C₁-C₆ alkyl group, a hydroxy C₁-C₆ alkyl group, a C₂-C₆ alkenyl group, an amino C₁-C₆ alkyl group, or a group of formula (III),
-(Aʺʺ)-Si(R₆")_{d"}(OR₅")_{c"} (III),
- c represents an integer from 1 to 3,
- d represents the integer 3 - c,
- c' represents an integer from 1 to 3,
- d' represents the integer 3 - c',
- c" represents an integer from 1 to 3,
- d" represents the integer 3 - c",
- e represents 0 or 1,
- f represents 0 or 1,
- g represents 0 or 1,
- h represents 0 or 1,
with the proviso that at least one of the functional groups e, f, g and h is different from 0.

3. The two-component system according to either claim 1 or claim 2,
**characterized in that**
the two-component system for treating a keratinous material contains at least one organosilicon compound of formula (I), which is selected from the group consisting of
- (3-aminopropyl)trimethoxysilane,
- (3-aminopropyl)triethoxysilane,
- (2-aminoethyl)trimethoxysilane, and
- (2-aminoethyl)triethoxysilane.

4. The two-component system according to either claim 2 or claim 3, **characterized in that** the two-component system contains at least one organosilicon compound of formula (II), which is selected from the group consisting of
- 3-(trimethoxysilyl)-N-[3-(trimethoxysilyl)propyl]-1-propanamine,
- 3-(triethoxysilyl)-N-[3-(triethoxysilyl)propyl]-1-propanamine,
- N-methyl-3-(trimethoxysilyl)-N-[3-(trimethoxysilyl)propyl]-1-propanamine,
- N-methyl-3-(triethoxysilyl)-N-[3-(triethoxysilyl)propyl]-1-propanamine
- 2-[bis[3-(trimethoxysilyl)propyl]amino]ethanol,
- 2-[bis[3-(triethoxysilyl)propyl]amino]ethanol,
- 3-(trimethoxysilyl)-N,N-bis[3-(trimethoxysilyl)propyl]-1-propanamine
- 3-(triethoxysilyl)-N,N-bis[3-(triethoxysilyl)propyl]-1-propanamine
- N1,N1-bis[3-(trimethoxysilyl)propyl]-1,2-ethanediamine,
- N1,N1-bis[3-(triethoxysilyl)propyl]-1,2-ethanediamine,
- N,N-bis[3-(trimethoxysilyl)propyl]-2-propen-1-amine, and
- N,N-bis[3-(triethoxysilyl)propyl]-2-propen-1-amine.

5. The two-component system according to one of claims 1 to 4, **characterized in that** the organosilicon compound is contained in the water-free carrier medium in an amount of from 0.01 to 10 wt.%, preferably from 0.02 to 9 wt.%, more preferably from 0.05 to 8 wt.%, most preferably from 0.1 to 7 wt.%, based on the total weight of the water-free carrier medium, the organosilicon compound being (3-aminopropyl)triethoxysilane in particular.

6. The two-component system according to one of claims 1 to 5, **characterized in that** the two-component system contains at least one organosilicon compound of formula (IV),
R₉Si(OR₁₀)ₖ(R₁₁)ₘ (IV),
which is selected from the group consisting of
- methyltrimethoxysilane,
- methyltriethoxysilane,
- ethyltrimethoxysilane,
- ethyltriethoxysilane,
- propyltrimethoxysilane,
- propyltriethoxysilane,
- hexyltrimethoxysilane,
- hexyltriethoxysilane,
- octyltrimethoxysilane,
- octyltriethoxysilane,
- dodecyltrimethoxysilane, and
- dodecyltriethoxysilane.

7. The two-component system according to one of claims 1 to 6, **characterized in that** the alkane is a C10-C24 alkane, preferably a C12-C18 alkane and more preferably a C14-C16 alkane, and/or the alkane being contained in the water-free carrier medium in an amount of 1 to 50 wt.%, preferably 2 to 45 wt.%, more preferably 3 to 40 wt.%, even more preferably 4 to 35 wt.%, based on the total weight of the water-free carrier medium, and/or **characterized in that**
the fatty alcohol is a C12-C24 fatty alcohol, preferably a C14-C18 fatty alcohol, and/or the fatty alcohol being contained in the water-free carrier medium in an amount of 5 to 50 wt.%, preferably 6 to 45 wt.%, more preferably 7 to 40 wt.%, even more preferably 8 to 35 wt.%, based on the total weight of the water-free carrier medium, and/or **characterized in that**
the alcohol is a C3-C6 alcohol, preferably a C4-C5 alcohol, and/or the alcohol being contained in the water-free carrier medium in an amount of 4 to 50 wt.%, preferably 6 to 45 wt.%, more preferably 8 to 40 wt.%, even more preferably 10 to 30 wt.%, based on the total weight of the water-free carrier medium and/or **characterized in that**
the branched or linear amine is a C3-C8, more preferably a C4-C6 amine, and/or the amine being contained in the water-free carrier in an amount of 0.5 to 10 wt.%, preferably 0.75 to 7.5 wt.%, more preferably 1 to 5 wt.%, based on the total weight of the water-free carrier.

8. The two-component system according to one of claims 1 to 7, **characterized in that** the aqueous phase or the water-free carrier medium comprises one or more emulsifiers,
the emulsifier being preferably an alkyltrimonium compound having one or more C8-C22, more preferably C10-C18, even more preferably C12-C16 alkyl groups, and/or the emulsifier being preferably a fatty alcohol ethoxylate in which the fatty alcohol portion of the fatty alcohol ethoxylate has an alkyl chain length of C4-C30, preferably C6-C25, more preferably C80-C20 and/or in which the number of ethoxy groups in the fatty alcohol ethoxylate is 2 to 120, preferably 4 to 100, more preferably 6 to 80, even more preferably 8 to 60, most preferably 10 to 40, and/or
the emulsifier being preferably a fatty alcohol sulfate having a chain length of C8-C22, preferably C10-C20, more preferably C12-C18.

9. The two-component system according to one of claims 1 to 8, **characterized in that** the alkyltrimonium compound is contained in the aqueous phase in an amount of 0.1 to 5 wt.%, preferably 0.5 to 4 wt.%, more preferably 1 to 3 wt.%, based on the total weight of the aqueous phase, or the alkyltrimonium compound is contained in the water-free carrier medium in an amount of 0.1 to 5 wt.%, preferably 0.5 to 4 wt.%, more preferably 1 to 3 wt.%, based on the total weight of the water-free carrier medium, and/or **characterized in that** the fatty alcohol ethoxylate is contained in the aqueous phase in an amount of 0.1 to 10 wt.%, preferably 0.25 to 7.5 wt.%, more preferably 0.5 to 5 wt.%, based on the total weight of the aqueous phase, or the fatty alcohol ethoxylate is contained in the water-free carrier medium in an amount of 0.1 to 10 wt.%, preferably 0.25 to 7.5 wt.%, more preferably 0.5 to 5 wt.%, based on the total weight of the water-free carrier medium, and/or **characterized in that** the fatty alcohol sulfate is contained in the aqueous phase in an amount of 0.1 to 10 wt.%, preferably 0.25 to 7.5 wt.%, more preferably 0.5 to 5 wt.%, based on the total weight of the aqueous phase, or the fatty alcohol sulfate is contained in the water-free carrier medium in an amount of 0.1 to 10 wt.%, preferably 0.25 to 7.5 wt.%, more preferably 0.5 to 5 wt.%, based on the total weight of the water-free carrier medium.

10. The two-component system according to one of claims 1 to 9, **characterized in that** the hydrogen peroxide is contained in the aqueous phase in an amount of 1 to 12 wt.%, preferably 2 to 10 wt.%, more preferably 3 to 8 wt.%, based on the total weight of the aqueous phase, and/or **characterized in that**
the dye for changing color is selected from the group consisting of
1,7-NAPHTHALENEDIOL, m-PHENYLENEDIAMINE, TOLUENE-2,5-DIAMINE SULFATE, 2,4-DIAMINOANISOL, p-PHENYLENEDIAMINE HCl, p-PHENYLENEDIAMINE (free base), 2-CHLORO-p-PHENYLENEDIAMINE, N-PHENYL-p-PHENYLENEDIAMINE, RESORCINOL, 4-CHLORORESORCINOL, o-AMINOPHENOL, m-AMINOPHENOL, p-AMINOPHENOL, 1-NAPHTHOL, 1,5-NAPHTHALENEDIOL, 2,7-NAPHTHENEDIOL, HYDROQUINONE, p-METHYLAMINOPHENOL, p-METHYLAMINOPHENOLSULFATE, HYDROXYBENZOMORPHOLINE, 4-AMINO-2-HYDROXYTOLUENE, 2-METHYL-5-HYDROXYETHYLAMINOPHENOL, 1,2,4-TRIHYDROXYBENZENE, PHENYL METHYL PYRAZOLONE, 2,4-DIAMINOPHENOXYETHANOL HCl or SO4, 3-AMINO-2,4-DICHLOROPHENOL HCl, 2-METHYLRESORCINOL, N,N-BIS(2-HYDROXYETHYL)-p-PHENYLENEDIAMINE SULFATE, TETRAAMINOPYRIMIDINE SULFATE, 4-AMINO-m-CRESOL, 6-AMINO-m-CRESOL, 1,3-BIS-(2,4-DIAMINOPHENOXY)PROPANE x 4 HCl, HYDROXYETHYL-p-PHENYLENEDIAMINE SULFATE, 2-AMINO-4-HYDROXYETHYLAMINOANISOLE SULFATE, 4,4-DIAMINODIPHENYLAMINE SULFATE, 5-AMINO-6-CHLORO-o-CRESOL, HYDROXYETHYL-3,4-METHYLENEDIOXYANILINE HCl, 2,6-DIHYDROXY-3,4-DIMETHYLPYRIDINE, 2,6-DIMETHOXY-3,5-PYRIDINEDIAMINE x (2) HCl, DIHYDROXYINDOLE, 2-AMINOMETHYL-p-AMINOPHENOL HCl, 2,4-DIAMINO-5-METHYLPHENETOL HCl, 5-AMINO-4-CHLORO-o-CRESOL HCl, HYDROXYPROPYL BIS(N-HYDROXYETHYL-p-PHENYLENEDIIMAMINE) HCl, 6-HYDROXYINDOLE, ISATIN, 6-METHOXY-2-METHYLAMINO-3-AMINOPYRIDINE HCl (HC BLUE 7), 2-AMINO-3-HYDROXYPYRIDINE, 2-DIMETHYLAMINO-5-AMINOPYRIDINE x2HCL, 6-METHOXY-2,3-PYRIDINEDIAMINE HCL, 2,6-DIAMINO PYRIDINE, 2,6-DIHYDROXYETHYLAMINOTOLUENE, 2,5,6-TRIAMINO-4_PYRIMIDINOL SULFATE, DIHYDROXYINDOLINE HBr, 1-ACETOXY-2-METHYLNAPHTHALENE, 1-HYDROXYETHYL 4,5-DIAMINOPYRAZOLE SULFATE, 2,2'-METHYLENEBIS 4-AMINOPHENOL HCl, 2-METHYL-1-NAPHTHOL, 4-formyl 1-methylquinolinium-p-toluene sulfonate, 2-AMINO-5-ETHYLPHENYL HCL, 2,3-DIAMINODIHYDROXY PYRAZOLOPYRAZOLONE DIMETHOSULFONATE, 2-METHOXY-METHYL-p-PHENYLENEDIAMINE, HYDROXYETHOXY AMINOPYRAZOLOPYRIDINE HCL, 3-AMINO-2,6-DIMETHYLPHENOL, 1-HEXYL 4,5-DIAMINO PYRAZOLE, SULFATE, DIMETHYLPIPERAZINIUM AMINOPYRAZOLO PYRIDINE, METHYLIMIDAZOLIUM p-PHENYLENEDIAMINE, ACID YELLOW 1, DISPERSE RED 17, BASIC BROWN 17, ACID BLACK 52, ACID BLACK 1, DISPERSE VIOLET 4, 4-NITRO-o-PHENYLENDIAMINE, 2-NITRO-P-PHENYLENEDIAMINE, 2-AMINO-4-NITROPHENOL, 2-AMINO-5-NITROPHENOL, PICRAMIC ACID and SODIUM PICRAMATE, HC RED NO. 13, N,N'-BIS(2-HYDROXYETHYL)-2-NITRO-p-PHENYLENEDIAMINE, HC YELLOW NO. 5, HC RED NO. 7, HC BLUE NO. 2, HC YELLOW NO. 4, HC YELLOW NO. 2, HC ORANGE NO. 1, HC RED NO. 1, HC RED NO. 3, 4-AMINO-3-NITROPHENOL, 2-HYDROXYETHYLAMINO-5-NITROANISOLE, 3-NITRO-p-HYDROXYETHYLAMINOPHENOL, 3-METHYLAMINO-4-NITROPHENOXYETHANOL, 2-NITRO-5-GLYCERYL METHYLANILINE, HC VIOLET NO. 1, HC ORANGE NO. 2, HC YELLOW NO. 9, 4-NITROPHENYLAMINOETHYLUREA, HC RED NO. 10 + HC RED NO. 11, 2-HYDROXYETHYL PICRAMIC ACID, HC BLUE NO. 12 as HCl, HC YELLOW NO. 6, HYDROXYETHYL-2-NITRO-p-TOLUIDINE, HC YELLOW NO. 12, HC BLUE NO. 11, HC YELLOW NO. 7, HC YELLOW NO. 10, 4-AMINO-2-NITRODIPHENYL-AMINE-2'-CARBOXYLIC ACID, 2-CHLORO-6-ETHYLAMINO-4-NITROPHENOL, HC VIOLET NO. 2, 2-AMINO-6-CHLORO-4-NITROPHENOL, 4-HYDROXYPROPYLAMINO-3-NITROPHENOL, HC YELLOW NO. 13, TETRAHYDRO-6-NITROQUINOXALINE, 2,6-DIAMINO-3-((PYRIDINE-3-YL)AZO)PYRIDINE, BASIC ORANGE 69, HC RED 16 / N-(2-Nitro-4-aminophenyl)-allylamine, BASIC VIOLET 2 as HCl, BASIC RED 51, BASIC YELLOW 87, BASIC ORANGE 31, HC BLUE 16, HC RED NO. 17, HC YELLOW 17, HC BLUE 18, HC YELLOW 16, HC RED 18, HC ORANGE 6, BASIC BLUE 124, BASIC RED 76, BASIC BROWN 16, BASIC YELLOW 57, ACID ORANGE 7, ACID RED 33, ACID YELLOW 23, ACID BLUE 9, ACID RED 92, "ACID YELLOW 3 (mono and di sodium)", BASIC BLUE 99, ACID VIOLET 43, DISPERSE VIOLET 1, DISPERSE BLUE 3, ACID BLUE 62, DISPERSE BLACK 9, HYDROXYANTHRAQUINONE AMINOPROPYL METHYL MORPHOLINIUM METHOSULFATE, LAWSONE, LAWSONIA INERMIS, INDIGOFERA TINCTORIA, HC BLUE NO. 14, CURRY RED, ACID RED 18, ACID RED 52, ACID GREEN 25, DISPERSE BLUE 377, PIGMENT RED 57, HC BLUE NO. 15, TETRABROMOPHENOL BLUE, HC BLUE NO. 17 or BISMUTH CITRATE.

11. The two-component system according to one of claims 1 to 10, **characterized in that** the weight ratio of the water-free carrier medium to the aqueous phase is in the range from 1 to 10 to 10 to 1, preferably 5 to 1 to 1 to 5, more preferably from 2 to 1 to 1 to 2.

12. The use of a two-component system according to one of claims 1 to 11 for coloring a keratinous material or for maintaining and coloring a keratinous material.

## Revendications

1. Système à deux composants pour la coloration artificielle de matière kératinique, comprenant, séparés l'un de l'autre
• un milieu de support anhydre comme premier composant, comprenant
a) au moins un alcane en C8-C30 ramifié ou linéaire,
b) au moins un alcool gras en C10-C30 ramifié ou linéaire,
c) au moins un alcool en C2-C8 monovalent, ramifié ou linéaire,
d) au moins une amine en C2-C10 ramifiée ou linéaire comme agent alcalinisant,
e) un ou plusieurs colorants destinés à modifier la couleur de la matière kératinique, et
f) au moins un composé organique de silicium, dans lequel l'au moins un composé organique de silicium contient un composé de formule (I) et au moins un composé organique de silicium de formule (IV),
où, dans le composé organique de silicium de formule (I)
R₁R₂N-L-Si(OR₃)ₐ(R₄)_{b} (I),
- R₁, R₂ représentent tous deux un atome d'hydrogène,
- L représente un groupe alkylène en C₁-C₆ linéaire divalent, de préférence un groupe propylène
(-CH₂-CH₂-CH₂-) ou un groupe éthylène (-CH₂-CH₂-),
- R₃, R₄ représentent, indépendamment l'un de l'autre, un groupe méthyle ou un groupe éthyle,
- a représente le nombre 3 et
- b représente le nombre 0, et
où, dans le composé organique de silicium de formule (IV)
R₉Si(OR₁₀)ₖ(R₁₁)ₘ (IV),
- R₉ représente un groupe alkyle en C₁-C₁₂,
- R₁₀ représente un atome d'hydrogène ou un groupe alkyle en C₁-C₆,
- R₁₁ représente un groupe alkyle en C₁-C₆,
- k représente un nombre entier allant de 1 à 3, et
- m représente le nombre entier 3 - k, et
• une phase aqueuse comme second composant, comprenant de l'eau et du peroxyde d'hydrogène.

2. Système à deux composants selon la revendication 1,
**caractérisé en ce que** le milieu de support anhydre comprend en outre au moins un composé organique de silicium qui représente un composé de formule (II),
où, dans le composé organique de silicium de formule (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} et R_{6"} représentent, indépendamment les uns des autres, un groupe alkyle en C₁-C₆,
- A, A', A", A‴ et Aʺʺ représentent, indépendamment les uns des autres, un groupe alkylène en C₁-C₂₀ divalent, linéaire ou ramifié,
- R₇ et R₈ représentent, indépendamment l'un de l'autre, un atome d'hydrogène, un groupe alkyle en C₁-C₆, un groupe hydroxy alkyle en C₁-C₆, un groupe alcényle en C₂-C₆, un groupe amino alkyle en C₁-C₆ ou un groupement de formule (III)
- (Aʺʺ)-Si(R₆")_{d"}(OR₅")_{c"} (III),
- c représente un nombre entier allant de 1 à 3,
- d représente le nombre entier 3 - c,
- c' représente un nombre entier allant de 1 à 3,
- d' représente le nombre entier 3 - c',
- c" représente un nombre entier allant de 1 à 3,
- d" représente le nombre entier 3 - c",
- e représente 0 ou 1,
- f représente 0 ou 1,
- g représente 0 ou 1,
- h représente 0 ou 1,
à condition qu'au moins l'un des radicaux parmi e, f, g et h soit différent de 0.

3. Système à deux composants selon l'une des revendications 1 ou 2,
**caractérisé en ce que**
le système à deux composants destiné à traiter une matière kératinique contient au moins un composé organique de silicium de formule (I) qui est choisi dans le groupe constitué de
- (3-aminopropyl)triméthoxysilane
- (3-aminopropyl)triéthoxysilane
- (2-aminoéthyl)triméthoxysilane et
- (2-aminoéthyl)triéthoxysilane.

4. Système à deux composants selon l'une des revendications 2 à 3, **caractérisé en ce que** le système à deux composants contient au moins un composé organique de silicium de formule (II) qui est choisi dans le groupe constitué de
- 3-(triméthoxysilyl)-N-[3-(triméthoxysilyl)propyl]-1-propanamine
- 3-(triéthoxysilyl)-N-[3-(triéthoxysilyl)propyl]-1-propanamine
- N-méthyl-3-(triméthoxysilyl)-N-[3-(triméthoxysilyl)propyl]-1-propanamine
- N-méthyl-3-(triéthoxysilyl)-N-[3-(triéthoxysilyl)propyl]-1-propanamine
- 2-[bis[3-(triméthoxysilyl)propyl]amino]-éthanol
- 2-[bis[3-(triéthoxysilyl)propyl]amino]-éthanol
- 3-(triméthoxysilyl)-N,N-bis[3-(triméthoxysilyl)propyl]-1-propanamine
- 3-(triéthoxysilyl)-N,N-bis[3-(triéthoxysilyl)propyl]-1-propanamine
- N1,N1-bis[3-(triméthoxysilyl)propyl]-1,2-éthanediamine,
- N1,N1-bis[3-(triéthoxysilyl)propyl]-1,2-éthanediamine,
- N,N-bis[3-(triméthoxysilyl)propyl]-2-propén-1-amine et
- N,N-bis[3-(triéthoxysilyl)propyl]-2-propén-1-amine.

5. Système à deux composants selon l'une des revendications 1 à 4, **caractérisé en ce que** le composé organique de silicium est contenu dans le milieu de support anhydre en une quantité allant de 0,01 à 10 % en poids, de préférence de 0,02 à 9 % en poids, plus préférablement de 0,05 à 8 % en poids, le plus préférablement de 0,1 à 7 % en poids, par rapport au poids total du milieu de support anhydre, dans lequel le composé organique de silicium est en particulier le (3-aminopropyl)triéthoxysilane.

6. Système à deux composants selon l'une des revendications 1 à 5, **caractérisé en ce que** le système à deux composants contient au moins un composé organique de silicium de formule (IV)
R₉Si(OR₁₀)ₖ(R₁₁)ₘ (IV),
lequel est choisi dans le groupe constitué de
- méthyltriméthoxysilane
- méthyltriéthoxysilane
- éthyltriméthoxysilane
- éthyltriéthoxysilane
- propyltriméthoxysilane
- propyltriéthoxysilane
- hexyltriméthoxysilane
- hexyltriéthoxysilane
- octyltriméthoxysilane
- octyltriéthoxysilane
- dodécyltriméthoxysilane et
- dodécyltriéthoxysilane.

7. Système à deux composants selon l'une des revendications 1 à 6, **caractérisé en ce que** l'alcane est un alcane en C10-C24, de préférence un alcane en C12-C18 et plus préférablement un alcane en C14-C16, et/ou dans lequel l'alcane est contenu dans le milieu de support anhydre en une quantité allant de 1 à 50 % en poids, de préférence de 2 à 45 % en poids, plus préférablement de 3 à 40 % en poids, encore plus préférablement de 4 à 35 % en poids, par rapport au poids total du milieu de support anhydre, **et/ou caractérisé en ce que**
l'alcool gras est un alcool gras en C12-C24, de préférence un alcool gras en C14-C18, et/ou dans lequel l'alcool gras est contenu dans le milieu de support anhydre en une quantité allant de 5 à 50 % en poids, de préférence de 6 à 45 % en poids, plus préférablement de 7 à 40 % en poids, encore plus préférablement de 8 à 35 % en poids, par rapport au poids total du milieu de support anhydre, **et/ou caractérisé en ce que**
l'alcool est un alcool en C3-C6, de préférence un alcool en C4-C5, et/ou dans lequel l'alcool est contenu dans le milieu de support anhydre en une quantité allant de 4 à 50 % en poids, de préférence de 6 à 45 % en poids, plus préférablement de 8 à 40 % en poids, encore plus préférablement de 10 à 30 % en poids, par rapport au poids total du milieu de support anhydre, **et/ou caractérisé en ce que**
l'amine ramifiée ou linéaire est une amine en C3-C8, de préférence une amine en C4-C6, et/ou dans lequel l'amine est contenue dans le support anhydre en une quantité allant de 0,5 à 10 % en poids, de préférence de 0,75 à 7,5 % en poids, plus préférablement de 1 à 5 % en poids, par rapport au poids total du support anhydre.

8. Système à deux composants selon l'une des revendications 1 à 7, **caractérisé en ce que** la phase aqueuse ou le milieu de support anhydre comprend un ou plusieurs émulsifiants,
dans lequel l'émulsifiant est de préférence un composé d'alkyltrimonium comportant un ou plusieurs groupes alkyle en C8-C22, plus préférablement en C10-C18, encore plus préférablement en C12-C16, et/ou dans lequel l'émulsifiant est de préférence un éthoxylate d'alcool gras, dans lequel la partie alcool gras de l'éthoxylate d'alcool gras présente une longueur de chaîne alkyle allant de C4 à C30, de préférence de C6 à C25, plus préférablement de C8 à C20, et/ou dans lequel le nombre de groupes éthoxy dans l'éthoxylate d'alcool gras va de 2 à 120, de préférence de 4 à 100, plus préférablement de 6 à 80, encore plus préférablement de 8 à 60, le plus préférablement de 10 à 40, et/ou
dans lequel l'émulsifiant est de préférence un sulfate d'alcool gras comportant une longueur de chaîne allant de C8 à C22, de préférence de C10 à C20, plus préférablement de C12 à C18.

9. Système à deux composants selon l'une des revendications 1 à 8, **caractérisé en ce que** le composé d'alkyltrimonium est contenu dans la phase aqueuse en une quantité allant de 0,1 à 5 % en poids, de préférence de 0,5 à 4 % en poids, plus préférablement de 1 à 3 % en poids, par rapport au poids total de la phase aqueuse, ou le composé d'alkyltrimonium est contenu dans le milieu de support anhydre en une quantité allant de 0,1 à 5 % en poids, de préférence de 0,5 à 4 % en poids, plus préférablement de 1 à 3 % en poids, par rapport au poids total du milieu de support anhydre, et/ou **caractérisé en ce que** l'éthoxylate d'alcool gras est contenu dans la phase aqueuse en une quantité allant de 0,1 à 10 % en poids, de préférence de 0,25 à 7,5 % en poids, plus préférablement de 0,5 à 5 % en poids, par rapport au poids total de la phase aqueuse, ou l'éthoxylate d'alcool gras est contenu dans le milieu de support anhydre en une quantité allant de 0,1 à 10 % en poids, de préférence de 0,25 à 7,5 % en poids, plus préférablement de 0,5 à 5 % en poids, par rapport au poids total du milieu de support anhydre, et/ou **caractérisé en ce que** le sulfate d'alcool gras est contenu dans la phase aqueuse en une quantité allant de 0,1 à 10 % en poids, de préférence de 0,25 à 7,5 % en poids, plus préférablement de 0,5 à 5 % en poids, par rapport au poids total de la phase aqueuse, ou le sulfate d'alcool gras est contenu dans le milieu de support anhydre en une quantité allant de 0,1 à 10 % en poids, de préférence de 0,25 à 7,5 % en poids, plus préférablement de 0,5 à 5 % en poids, par rapport au poids total du milieu de support anhydre.

10. Système à deux composants selon l'une des revendications 1 à 9, **caractérisé en ce que** le peroxyde d'hydrogène est contenu dans la phase aqueuse en une quantité allant de 1 à 12 % en poids, de préférence de 2 à 10 % en poids, plus préférablement de 3 à 8 % en poids, par rapport au poids total de la phase aqueuse, **et/ou caractérisé en ce que**
le colorant destiné à modifier la couleur est choisi dans le groupe constitué de
1,7-NAPHTHALÈNEDIOL, m-PHÉNYLÈNEDIAMINE, TOLUÈNE-2,5-DIAMINE SULFATE, 2,4-DIAMINOANISOL, p-PHÉNYLÈNEDIAMINE HCl, p-PHÉNYLÈNEDIAMINE (base libre), 2-CHLORO-p-PHÉNYLÈNEDIAMINE, N-PHÉNYL-p-PHÉNYLÈNEDIAMINE, RÉSORCINOL, 4-CHLORORÉSORCINOL, o-AMINOPHÉNOL, m-AMINOPHÉNOL, p-AMINOPHÉNOL, 1-NAPHTOL, 1,5-NAPHTHALÈNEDIOL, 2,7-NAPHTHALÈNEDIOL, HYDROQUINONE, p-MÉTHYLAMINOPHÉNOL, p-MÉTHYLAMINOPHÉNOLSULFATE, HYDROXYBENZOMORPHOLINE, 4-AMINO-2-HYDROXYTOLUÈNE, 2-MÉTHYL-5-HYDROXYÉTHYLAMINOPHÉNOL, 1,2,4-TRIHYDROXYBENZÈNE, PHÉNYL MÉTHYL PYRAZOLONE, 2,4-DIAMINOPHÉNOXYÉTHANOL HCl ou SO4, 3-AMINO-2,4-DICHLOROPHÉNOL HCl, 2-MÉTHYLRÉSORCINOL, N,N-BIS(2-HYDROXYÉTHYL)-p-PHÉNYLÈNEDIAMINE SULFATE, TÉTRAAMINOPYRIMIDINE SULFATE, 4-AMINO-m-CRÉSOL, 6-AMINO-m-CRÉSOL, 1,3-BIS-(2,4-DIAMINOPHÉNOXY)PROPANE x 4 HCl, HYDROXYÉTHYL-p-PHÉNYLÈNEDIAMINE SULFATE, 2-AMINO-4-HYDROXYÉTHYLAMINOANISOLE SULFATE, 4,4-DIAMINODIPHÉNYLAMINE SULFATE, 5-AMINO-6-CHLORO-o-CRÉSOL, HYDROXYÉTHYL-3,4-MÉTHYLÈNEDIOXYANILINE HCl, 2,6-DIHYDROXY-3,4-DIMÉTHYLPYRIDINE, 2,6-DIMÉTHOXY-3,5-PYRIDINEDIAMIE x (2) HCl, DIHYDROXYINDOLE, 2-AMINOMÉTHYL-p-AMINOPHÉNOL HCl, 2,4-DIAMINO-5-MÉTHYLPHÉNÉTOLE HCl, 5-AMINO-4-CHLORO-o-CRÉSOL HCl, HYDROXYPROPYL BIS(N-HYDROXYÉTHYL-p-PHÉNYLÈNEDIMAMINE) HCl, 6-HYDROXYINDOLE, ISATINE, 6-MÉTOXY-2-MÉTHYLAMINO-3-AMINOPYRIDINE HCl (HC BLUE 7), 2-AMINO-3-HYDROXYPYRIDINE, 2-DIMÉTHYLAMINO-5-AMINOPYRIDINE x2HCL, 6-MÉTHOXY-2,3-PYRIDINEDIAMINE HCL, 2,6-DIAMINO PYRIDINE, 2,6-DIHYDROXYÉTHYLAMINOTOLUÈNE, 2,5,6-TRIAMINO-4_PYRIMIDINOL SULFATE, DIHYDROXYINDOLINE HBr, 1-ACÉTOXY-2-MÉTHYLNAPHTHALÈNE, 1-HYDROXYÉTHYL 4,5-DIAMINOPYRAZOLE SULFATE, 2,2'-MÉTHYLÈNEBIS 4-AMINOPHÉNOL HCl, 2-MÉTHYL-1-NAPHTHOL, 4-formyl 1-méthylquinolinium-p-toluène sulfonate, 2-AMINO-5-ÉTHYLPHÉNYL HCL, 2,3-DIAMINODIHYDROXY PYRAZOLOPYRAZOLONE DIMÉTHOSULFONATE, 2-MÉTHOXY-MÉTHYL-p-PHÉNYLÈNEDIAMINE, HYDROXYÉTHOXY AMINOPYRAZOLOPYRIDINE HCL, 3-AMINO-2,6-DIMÉTHYLPHÉNOL, 1-HEXYL 4,5-DIAMINO PYRAZOLE, SULFATE, DIMÉTHYLPIPÉRAZINIUM AMINOPYRAZOLOPYRIDINE, MÉTHYLIMIDAZOLIUM p-PHÉNYLÈNEDIAMINE, ACID YELLOW 1, DISPERSE RED 17, BASIC BROWN 17, ACID BLACK 52, ACID BLACK 1, DISPERSE VIOLET 4, 4-NITRO-o-PHÉNYLÈNEDIAMINE, 2-NITRO-P-PHÉNYLÈNEDIAMINE, 2-AMINO-4-NITROPHÉNOL, 2-AMINO-5-NITROPHÉNOL, ACIDE PICRAMIQUE et PICRAMATE DE SODIUM, HC RED NO. 13, N,N'-BIS(2-HYDROXYÉTHYL)-2-NITRO-p-PHÉNYLÈNEDIAMINE, HC YELLOW NO. 5, HC RED NO. 7, HC BLUE NO. 2, HC YELLOW NO. 4, HC YELLOW NO. 2, HC ORANGE NO. 1, HC RED NO. 1, HC RED NO. 3, 4-AMINO-3-NITROPHÉNOL, 2-HYDROXYÉTHYLAMINO-5-NITROANISOLE, 3-NITRO-p-HYDROXYÉTHYLAMINOPHÉNOL, 3-MÉTHYLAMINO-4-NITROPHÉNOXYÉTHANOL, 2-NITRO-5-GLYCÉRYL MÉTHYLANILINE, HC VIOLET NO. 1, HC ORANGE NO. 2, HC YELLOW NO. 9, 4-NITROPHÉNYLAMINOÉTHYLURÉE, HC RED NO. 10 + HC RED NO. 11, 2-HYDROXYÉTHYL ACIDE PICRAMIQUE, HC BLUE NO. 12 en tant que HCl, HC YELLOW NO. 6, HYDROXYÉTHYLE-2-NITRO-p-TOLUIDINE, HC YELLOW NO. 12, HC BLUE NO. 11, HC YELLOW NO. 7, HC YELLOW NO. 10, 4-AMINO-2-NITRO-DIPHÉNYL-AMINE-2'-ACIDE CARBOXYLIQUE, 2-CHLORO-6-ÉTHYLAMINO-4-NITROPHÉNOL, HC VIOLET NO. 2, 2-AMINO-6-CHLORO-4-NITROPHÉNOL, 4-HYDROXYPROPYLAMINO-3-NITROPHÉNOL, HC YELLOW NO. 13, TÉTRAHYDRO-6-NITROQUINOXALINE, 2,6-DIAMINO-3-((PYRIDIN-3-YL)AZO)PYRIDINE, BASIC ORANGE 69, HC RED 16/N-(2-nitro-4-aminophényl)-allylamine, BASIC VIOLET 2 en tant que HCl, BASIC RED 51, BASIC YELLOW 87, BASIC ORANGE 31, HC BLUE 16, HC RED NO. 17, HC YELLOW 17, HC BLUE 18, HC YELLOW 16, HC RED 18, HC ORANGE 6, BASIC BLUE 124, BASIC RED 76, BASIC BROWN 16, BASIC YELLOW 57, ACID ORANGE 7, ACID RED 33, ACID YELLOW 23, ACID BLUE 9, ACID RED 92, « ACID YELLOW 3 (monosodium et disodium) », BASIC BLUE 99, ACID VIOLET 43, DISPERSE VIOLET 1, DISPERSE BLUE 3, ACID BLUE 62, DISPERSE BLACK 9, HYDROXYANTHRAQUINONE AMINOPROPYL MÉTHYL MORPHOLINIUM MÉTHOSULFATE, LAWSONE, LAWSONIA INERMIS, INDIGOFERA TINCTORIA, HC BLUE NO. 14, CURRY RED, ACID RED 18, ACID RED 52, ACID GREEN 25, DISPERSE BLUE 377, PIGMENT RED 57, HC BLUE NO. 15, TÉTRABROMOPHÉNOL BLUE, HC BLUE NO. 17 ou BISMUTH CITRATE.

11. Système à deux composants selon l'une des revendications 1 à 10, **caractérisé en ce que** le rapport pondéral du milieu de support anhydre à la phase aqueuse se situe dans la plage allant de 1 pour 10 à 10 pour 1, de préférence de 5 pour 1 à 1 pour 5, plus préférablement de 2 pour 1 à 1 pour 2.

12. Utilisation d'un système à deux composants selon l'une des revendications 1 à 11 pour la coloration d'une matière kératinique ou pour le soin et la coloration d'une matière kératinique.
